Europäisches Patentamt

(19) European Patent Office

Office européen des brevets

(11) EP 0 511 317 B1

(12) EUROPEAN PATENT SPECIFICATION

(45) Date of publication and mention
of the grant of the patent:
**22.04.1998 Bulletin 1998/17**

(21) Application number: **91904854.6**

(22) Date of filing: **17.01.1991**

(51) Int. Cl.⁶: $A01N\ 1/02$

(86) International application number:
**PCT/US91/00351**

(87) International publication number:
**WO 91/10361 (25.07.1991 Gazette 1991/17)**

(54) **COMPOSITION TO IMPROVE SURVIVAL OF BIOLOGICAL MATERIALS**

ZUSAMMENSETZUNG ZUR VERBESSERUNG DER LEBENSERHALTUNG BIOLOGISCHER
MATERIALIEN

COMPOSITION AMELIORANT LA SURVIE DE MATERIAUX BIOLOGIQUES

(84) Designated Contracting States:
**AT BE CH DE DK ES FR GB GR IT LI LU NL SE**

(30) Priority: **17.01.1990 US 466050**
**03.08.1990 US 562461**

(43) Date of publication of application:
**04.11.1992 Bulletin 1992/45**

(73) Proprietor:
**THE REGENTS OF THE UNIVERSITY OF
CALIFORNIA
Oakland, CA 94612-3550 (US)**

(72) Inventors:
 • **RUBINSKY, Boris
 Albany, CA 94707 (US)**
 • **DEVRIES, Arthur L.
 Urbana, IL 61801 (US)**

(74) Representative:
**Harrison, David Christopher et al
MEWBURN ELLIS
York House
23 Kingsway
London WC2B 6HP (GB)**

(56) References cited:
 **WO-A-90/13571** **US-A- 4 565 643**

 • JOURNAL OF PLANT PHYSIOLOGY vol. 135,
 1989, DE pages 351 - 354 A.J.CUTLER ET. AL.
 'Winter flounder antifreeze protein improves the
 cold hardiness of plant tissues'
 • CRYOBIOLOGY vol. 13, no. 3, June 1976, NEW
 YORK, USA pages 334 - 340 Y.LIN ET. AL.
 'Compartmentalisation of NaCl in Frozen
 Solutions of Antifreeze Glycoproteins'
 • PROCEEDINGS OF THE NATIONAL ACADEMY
 OF SCIENCES OF THE USA vol. 86, no. 3,
 February 1989, USA pages 881 - 885
 J.A.RAYMOND ET. AL. 'Inhibition of growth of
 non-basal planes in ice by fish antifreezes'
 • CHEMICAL PATENTS INDEX, BASIC
 ABSTRACTS JOURNAL Section Ch, Week 8602,
 Derwent Publications Ltd., London, GB; Class C,
 AN 011139
 • CHEMICAL PATENTS INDEX, BASIC
 ABSTRACTS JOURNAL Section Ch, Week 8602,
 Derwent Publications Ltd., London, GB; Class C,
 AN 011139
 • Canadian Journal of Zoology (1986), volume 64,
 number 9

## Description

The present invention relates to the use of antifreeze protein or glycoprotein, hereinafter referred to as thermal hysteresis protein, which is derived, for example, from the fluid or serum of certain Arctic and Antarctic fish in a method for the protection and preservation of the viability of biological material selected from mammalian cells, tissues or organs. Preferred thermal hysteresis proteins are polypeptides having multiple -alanine-alanine-threonine- or -alanine-alanine-alanine-segments. In some embodiments, a pendant sugar group is covalently attached to the threonine moiety.

A solution of antifreeze protein is perfused through the biological material. The cells, tissue or organs is then carefully frozen to temperatures below -0.5°C and held at the low temperatures. The ice forms primarily along the c-axis of the ice crystal, and ice formation is inhibited in the direction of the a-axes (faces) of the ice crystal. This spicular ice growth compartmentalizes the concentration of the salts with the result that adjacent cells are not disrupted or completely dehydrated. The cells, tissue or organ is carefully thawed, and is functioning and viable. The preserved organs are particularly useful in transplantation therapy in human being.

The present invention also relates to the use of compositions containing the said thermal hysteresis proteins in a method to improve survival, functionality and/or structural integrity in said biological materials, exposed to temperatures different from their normal physiological temperatures by protecting at least the cell membranes from damage and cell contents from leakage due to exposure to nonphysiological thermal conditions.

The preservation of viable animal tissue, animal organs and living animals has been the subject of recent intense laboratory and medical research. Human organ transplants of heart, kidney, lung, liver and the like are now possible because of improved surgical techniques, improved anti-rejection drugs, and immediate availability of donated organs. Presently, donor organs are removed from a donor, cooled, stored on wet ice, but not frozen and within a maximum of a few hours are surgically placed in a recipient's body.

The preservation of animal tissue, animal organs and intact viable animals by freezing at lowered temperatures is presently limited to a few hours, because the normal formation of ice in an organ produces localized concentrated salt solutions. Water migrates from the nearby cells irreversibly dehydrating the cell. It is a major problem that these events disrupt the organ structure, and the organ does not reactivate upon thawing.

Advances in the development of immunosuppressants, improvements in organ transplantation techniques and the successful use of freezing for long-term preservation of cells have motivated intensive research efforts on methods for long-term preservation of biological organs through freezing. Recently, B. Rubinsky, U.S. Patent 4,531,373 disclosed an experimental technique using a directional solidification stage and low temperature scanning electron microscopy to facilitate the study the process of freezing in biological tissues.

B. Rubinsky et al., (1988) Proceedings of the Royal Society London, B., Vol. 234, pp. 343-358), also describes experimental results and a mathematical model for the freezing process and the mechanism of damage in biological tissue and biological organs.

None of the available literature below disclose a composition or a method to preserve for long times frozen tissue, organs or whole animals.

Earlier experimental results show that single, continuous ice crystals normally form along the blood vessels of frozen tissue. B. Rubinsky et al. (1988), Cryo-Letters, Vol. 8, p. 370; B. Rubinsky et al. (1988), Proc. Royal Soc. Lond., B234, 343. The structure of the frozen tissue depends on the cooling rate, (i.e., the temperature variation per unit time) during freezing. When tissue, such as liver, is frozen with low cooling rates (about 1°C/min to about 10°C/min), the smaller blood vessels (sinusoids) expand relative to those of the unfrozen normal liver tissue. In addition, the cells (hepatocytes) adjacent to the expanded sinusoids, are dehydrated without intracellular ice forming. However, at higher cooling rates, intracellular ice forms in the cells (hepatocytes) resulting in a reduced expansion of the sinusoids.

One explanation for the observed formation of continuous ice crystals along the blood vessels, for the expansion of the frozen blood vessels, and for the formation of intracellular ice during freezing with higher cooling rates is that ice formed in the vascular system does not propagate through the cell membranes or the blood vessel wall. Instead, ice forms within and propagates along the blood vessels where there is no barrier to the ice crystal growth process. Water in the cells surrounding the frozen blood vessels, being compartmentalized in small volumes, will, at first, remain supercooled. As the intravascular ice forms, water is removed from the solution in the vascular space, rendering the remaining solution hypertonic (higher in salts concentration). This higher concentration of solutes causes water to migrate irreversibly from the surrounding cells, through the semi-permeable cell membrane, into the blood vessel in order to equilibrate the difference in chemical potential. Consequently, the cells surrounding the blood vessel will dehydrate, and the water that leaves the cell then freezes in the vascular system. Water transport from cells through the cell membrane into the blood vessel, is a rate-governed process, which depends on the permeability of the cell membrane. Therefore, when larger organs are frozen using higher (i.e. faster) cooling rates, sufficient water remains in the cell for intracellular ice to form prior to the complete dehydration of the cell. A more detailed description of the process of freezing and a mathematical model that supports this description is found in the Rubinsky, et al. (1988) reference above. This result also leads to the conclusion that one of the possible modes of damage to frozen tissue is the observed expansion of the blood vessels which causes the disruption of the structural (mechanical) integrity of the organ. This mode of damage apparently does not affect cells frozen in suspensions, and may explain why organs do not survive freezing under the same conditions in which cells in suspensions survive.

The normal patterns of ice formation, in which the energetically preferred direction of ice growth is also the a-axes (prism face) of the hexagonal prism ice crystal, governs the process of freezing in tissue. Any hexagonal prism facet of the a-axes of the three-dimensional ice crystal has the same energetic preference and, therefore, during freezing of tissue, the ice crystal can continuously follow and grow along the blood vessel. Furthermore, as discussed earlier, the large ice crystals of normal freezing do not incorporate solutes. This rejection of solutes results in more concentrated solutes, a mass transfer process and the irreversible water migration from local cells and tissue into the open vessel. This migration leads to the disruption of the structural integrity of the cells of the tissue or organ.

Additional background information can be found in:

B. Rubinsky et al., Biochem. Biophys. Res. Commun., Vol. 173, # 3, Dec. 1990, p. 1369-1374.
N.V. Jamieson et al. Cryobiology, Vol. 25, 300-310 (1988)
R.L. Veech, U.S. Patent No. 4,663,289.
C.A. Knight, et al. (1989), Science, Vol. 245, Aug. 4, 1989, pp. 505-507.
J.A. Raymond, et al. (1989), Proceedings of the National Academy of Sciences U.S.A., Vol. 86, pp. 881-885.
D.A. Powers (1989), Science, Vol. 246, October 20, 1989, pp. 352-358.
A.L. DeVries, (1984) Phil. Trans. R. Soc. London, Vol. B 304, pp. 575-588.
C.-H.C. Cheng, et al. (1989), Biochemical et Biophysical Acta, Vol. 997, pp. 55-64.
W. Wippich, German Patentsschrift 139, 200, Dec. 19, 1979.
J.D. Schrag, et al. (1987) Biochemical et Biophysical Acta, Vol. 915, pp. 357-370.
C.A. Knight et al., (1984) Nature, Vol. 308, no. 5956, pp. 295-296.
J.A. Raymond, et al., (1977) Proc. Nat. Acad. Sci. U.S.A., Vol. 74, no. 6, pp. 2589-2593.
J.A. Ahlgren, et al., (1988) J. Exp. Biol., Vol. 137, pp. 549-563.
P. Mazur, (1963) J. Gen. Physiol., Vol. 47, p. 347.
P. Mazur, (1970) Science, Vol. 168, p. 939.
A.L. DeVries, et al. (1970) J. Biol. Chem., Vol. 245, pp 2901-2908.
J.A. Raymond, et al. (1977) Proc. Natl. Acad. Sci. USA Vol. 74, pp. 2589-2593.
A.L. DeVries (1984) Trans. Royal Soc. Lond., Vol. B304, pp. 575-588.
A.L. DeVries, (1988) Comp. Biochem. Physiol. Vol. 90B (3) pp. 611-621.
J.A. Raymond, et al. (1989) Proc. Natl. Acad. Sci., Vol. 86(3), pp. 881-885.
H. Niemann, (1985) Theriogenology, Vol. 23 pp. 213.
M.L. Fahning, et al. (1989) Cryobiology, Vol. 26, pp. 563.
S. Hayashi, et al. (1989) The Vet. Rec., pp. 43-44.
C.C. Cheng, et al. (1989) Biochem. Biophy. Acta., Vol. 997, pp. 55-64.
G.M. Fahy, et al. (1984) Cryobiology, Vol. 21 pp. 407-426.
W.F. Rall, et al. (1985) Nature, Vol. 313, pp. 573-575.
A. Trounson (1986) Fertility and Sterility, Vol. 46, pp. 1-12.
W.F. Rall, (1987) Cryobiology, Vol. 24, pp. 387-402.
J.M. Shaw, et al. (1989) Cryobiology, Vol. 26, pp. 413-421.
G.M. Fahy (1990), Scientific American, Vol. 262, pp. 20.
A. Arav, et al. (1990), Proc. 28th Annual Meeting Soc. for Cryobiology, Abstracts 42,43.
D. Turnbull (1969) Contemp. Phys. Vol. 10, pp. 473-488.
B. Rubinsky, U. S. Patent 4,531,373, July 1985.
B. Rubinsky, et al. (1985) Cryobiology, Vol. 22, pp. 55-62.
M. Mattioli, et al., (1988) Gamete Research, Vol. 21, pp. 223-232.
A. Arav, et al. (1988) Cryobiology, Vol. 25, pp. 567.
P. Quin, et al. (1982) J. Reprod. Fert., Vol. 66, pp. 161-168.

Cutler et al., J. Plant Physiol, (1989), **135**, 351-354 described the effect of antifreeze protein isolated from winter flounder on plant tissues. The depression of the freezing point of serum from three Antarctic fish was investigated by De Vries et al., Science, (1969), **163**, 1073-1075. The properties of solutions of antifreeze glycoproteins and sodium chloride were reported By Lin et al., in Cryobiology, (1976), **13**, 334-340. Additionally, the use of antifreeze peptides in conferring freezing resistance on live fish is described By Fletcher et al., Canadian Journal of Zoology, (1986), **64**, 1897-1901.

It would be extremely advantageous to have a method which would alter the preservation process of biological liquids in mammalian cell tissue. Thus, when the frozen cells, tissue or organs is carefully thawed, it results in viable cells, tissue or organs. The present invention provides such a preservation composition and method.

The present invention provides a method for the protection and preservation of the viability of biological material selected from mammalian cell, tissue or organs undergoing exposure to a non-physiological temperature; said method comprising contacting said biological material with a biologically compatible solution of at least one thermal hysteresis protein before exposure to said non-physiological temperature, wherein said thermal hysteresis protein is obtainable from Antarctic notothenoids, Channichthyidae, Bathydrconidae, Northern ocean gadoids, Pleuroectinae such as righteye flounders, cottids, and Zoarcoidel (eel pouts).

In a preferred embodiment, the present invention relates to the use of a thermal hysteresis protein in the above described method for improving survival, functionality, stability and structural integrity of biological materials selected from cells, tissue or organs, subjected to temperatures lower than 0°C in the presence of ice crystals:

(a) by modification of the structure of the ice crystals in the immediate vicinity of the biological materials;
(b) by reducing the number and the size of the ice crystals or by completely eliminating the ice crystals in the immediate vicinity of the biological materials; or
(c) by modifying the mode in which solutes are rejected by the ice formation and thereby changing the chemical composition of the solution surrounding the biological materials.

The thermal hysteresis protein is suitably used in the form of a biologically compatible aqueous solution. The thermal hysteresis protein is a macromolecule obtainable from fish preferably from Arctic, Antarctic, North Temperate or South Temperate Zones. More preferably, the protein is from body fluids (eg blood) from Antarctic fish, eg from the family Nototheniidae, including the species D. nawsoni and P. borchgrevinki or the Antarctic eel pout Rhigophila dearborni, or the Arctic winter flounder. All these antifreeze proteins are known and have the common property that they modify the structure of ice crystals.

In one embodiment, the thermal hysteresis protein is selected from a polypeptide, a glycopeptide, a polypeptide covalently bonded to biologically acceptable carrier, a glycopolypeptide covalently bonded to a carrier or mixtures thereof.

The thermal hysteresis protein suitably promotes ice crystal growth along the c-axis of the ice crystal, and inhibits growth of an ice crystal along the a-axis of the ice crystal. In another embodiment, the at least one type of thermal hysteresis protein has alternating hydrophobic regions and hydrophilic regions which repeat between each 16-17 or 19-20 Angstroms, or preferably repeat about between about each 16.5 or 19.5 Angstroms.

In one embodiment, the aqueous composition further includes additional preserving, protecting or vitrifying compounds selected from glycerol, dimethylsulfoxide, ethylene glycol, polyvinylpyrrolidone, glucose, sucrose, propanediol, propylene, glycol, carboxymethyl cellulose, or mixtures of these compounds which are known to protect cells and biological materials against freezing damage or to promote vitrification.

In preferred embodiment the present invention relates to a method for preservation, survival, functionality, stability and structure or integrity of biological materials selected from mammalian cells, tissues or organs, at non-physiological temperatures which method comprises:

(a) bringing the moiety to be preserved in contact with a biologically acceptable solution of a thermal hysteresis protein in sufficient concentration to interact with all the microorganisms, tissues or organs;
(b) exposure to the nonphysiological temperature;
(c) optionally first removing the thermal hysteresis protein;
(d) returning the cells, tissues or organs to a physiological temperature while optionally simultaneously removing the thermal hysteresis protein; or optionally
(e) subsequently removing the thermal hysteresis protein after returning the biological material to the physiological temperature.

In one embodiment, the temperatures are hypothermic, ie, close to 0°C or lower and are used for preservation of mammalian cells, tissues or organs. For example, pig oocytes are preserved in such a way at about 4°C to 24 hr or more. Rat livers are preserved by this method at 4°C for 24 hr or more.

In another aspect the present invention related to a method for preservation of mammalian cells, tissues or organs, at temperatures below 0°C to about -269°C (4K) which method comprises:

(a) bringing the moiety to preserved in contact with a biologically compatibly composition comprising at least one thermal hysteresis protein, for example in an aqueous solution, and optionally additional cryoprotective compounds such as glycerol, propylene glycol, etc;
(b) cooling preferably to cryogenic temperatures (by such means as liquid nigrogen) and either vitrifying or freezing the system according to the various concentrations and cooling rates using higher concentrations of the additional compounds, such as propylene glycol or glycerol and higher cooling rates which lead to vitrification and to lower freezing temperatures (e.g. with 40% v/v propyleneglycol/water) and with a cooling rate of 1,750°C/min., vitrification is achieved);

(c) maintaining the cells, tissues or organs, at these temperatures for periods of up to 24 hours, 7 days, 52 weeks or more than 10 years,

(d) warming, by such means as warm fluids or microwave heating, to physiological conditions, and

(e) removing the said biologically compatible composition, (eg by perfusion or flushing) and replacing them with physiological compatible solutions to regenerate the viable biological moiety.

For example, with 12.5% v/v propylene glycol/water at a cooling rate of 1,200°C/min., ice crystals were formed. In all cases, viable mouse embryos and pig oocytes were obtained after exposure to -130°C for several hours;

Pig oocytes, pig embryos and mouse embryos survive this protocol in a aqueous composition of 20 mg/ml antifreeze glycoproteins from Antarctic fish from the family Nothotheniidae.

BRIEF DESCRIPTION OF THE DRAWINGS

Figures 1A, 1B and 1C are transmission light micrographs of ice crystals (i), in aqueous solution is frozen with a cooling rate of 4°C/min on a directional solidification stage, see U.S. Patent 4,531,373.

Figures 2A, 2B and 2C are scanning electron micrographs of liver tissue perfused with 40 mg/ml AFGPs (see definitions below) and frozen with a cooling rate of approximately 4000°C/min.

Figures 3A, 3B, 3C, 3D and 3E are scanning electron micrographs of frozen liver tissue.

Fig. 4 (4A to 4D) are photographs of the cryopreservation of immature pig oocytes.

Fig. 5 (5A to 5B) are photographs of the cryopreservation of pig embryos at the two-cell stage.

Fig. 6 (6A, 6B and 6C) are photographs concerned with cryopreservation of mouse embryos at the two-cell stage.

Fig. 7 (7A, 7B and 7C) are photographs showing with the hypothermic preservation of pig oocytes.

Figure 8 is a photographic representation of rat liver tissue (A7) at a magnification of about x 400. This tissue was cooled to -35°C at 21.5°C/min.

Figure 9 is a photographic representation of rat liver tissue at a magnification of about x 400. This liver tissue was flushed with a Krebs solution containing 20 mg/ml of fractions 1-8 (Table 1) at 37°C prior to cooling to -35° at 21.5°C/min.

Figure 10 is a graph of the bile production from whole rat liver treated with Krebs solution thermal hysteresis protein as a function of time (see Example 6A).

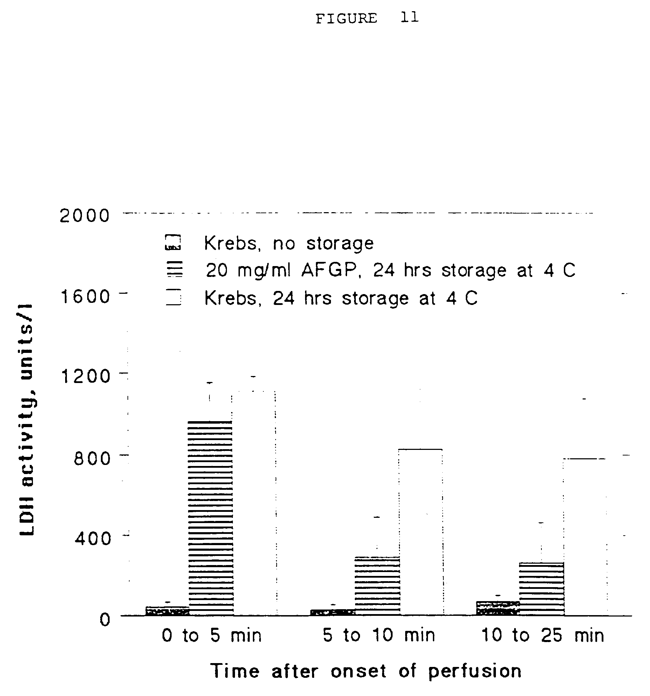

Figure 11 is a graph of the LDH level from whole rat liver treated with Krebs solution and Krebs solution and thermal hysteresis protein as a function of time (see Example 6A).

Figure 12 in a graphic representation of the percentage of oocytes with normal membrane potential after hypothermic exposure for 4 hr at 4°C without and with various concentration thermal hysteresis proteins.

Figure 13 is a graphic representation of the percentage of oocytes with normal membrane potential after hypothermic exposure for 24 hr at 4°C without and with various concentrations of thermal hysteresis proteins.

As used herein:

"Nonphysiological temperatures" refers to temperatures different from the normal physiological temperatures including high or lowered temperature or freezing.

As used herein the expression "Thermal hysteresis proteins" refers to peptides or proteins or glycopeptides or glycoproteins which act as antifreeze compounds. These are otherwise known as "antifreeze proteins" or "antifreeze peptides" which may be referred to herein as "AFP's" or "antifreeze glycopeptides" or "antifreeze glycoproteins" which will be referred to hereinafter to "AFGP's". These proteins are macromolecules found in the body fluids of some animals (e.g. cold blooded) which have the commonly known property that they reduce non-colligatively the phase transition temperature of water by direct interaction with and inhibition of the growth of ice crystal nucleii that form at temperatures below the phase transition temperature.

They are known as "thermal hysteresis proteins" because while the phase transition temperature is apparently depressed during freezing by an amount much larger than the colligative effect of the molecule, it is not depressed during melting except to the extent caused by the colligative effect of the molecule. Prior to the present invention, this was the only known property of these antifreeze compounds. (Sources of antifreeze peptide (or protein) are described below

"Cryogenic temperatures" refers in the area of cryobiology, below 0°C to as low as 4K or lower.

"Hyperthermic" refers to temperatures higher than the normal physiological temperature of a cell, tissue, organ, plant or animal.

"Hypothermic" refers to temperatures lower than the normal physiological temperature of a cell, tissue, organ, plant or animal.

"Optional" or "optionally" refers to the situation in which a component may or may not be present, or where a step may or may not be performed, within the scope of the invention.

"Prism planes" refer to another convention to describe the growing ice formation on an ice crystal. There exist secondary prism planes perpendicular to the a-axes and pyramidal planes that project off these planes. Crystallography terminology describes these planes in terms of the following pyramidal Miller-Bravais indices:

| Primary prism plane | $(1\ 0\ \bar{1}\ 0)$ |
| Secondary prism plane | $(1\ 1\ \bar{2}\ 0)$ |
| Pyramidal pane from the primary prism plane | $(2\ 0\ \bar{2}\ \bar{1})$ |
| Pyramidal plane from the secondary prism plane | $(1\ 1\ \bar{2}\ 1)$ |

Ice crystal growth under normal circumstances is along the a-axes. Ice crystal growth using the AFPs or AFGPs of the present invention is altered to be preferred in the direction of the c-axis.

For more information, see Peter V. Hobbs (1974) Ice Physics, Clarendon Press, Oxford, England, Appendix A etc., p. 725 ff.

"Rapid cooling" refers to a technique developed for long term preservation of cells and biological organs at cryogenic temperatures. The rapid cooling is used to produce very small, non-damaging ice crystals, see A. Trounson, (1986) Fertility and Sterility, Vol. 46, 1-12.

"Vitrification" refers to a technique for long term preservation of cells and biological organs at cryogenic temperatures. The technique involves introduction into the biological materials of different cryoprotective compounds such as glycerol, dimethylsulfoxide, propylene glycol, etc. which depress colligatively the phase transition temperature for water and increase its temperature. Next, the whole cell suspension or organ is rapidly cooled in the presence of the cryoprotective compounds with the expectation that the water in the biological materials will remain polymorphous in a glass form and that no damaging ice crystals will occur. (See Fahy, G.M. et al., Cryobiology, Vol. 21, 407-426, (1984), W.F., Rall and Rahy, G.M. Nature, Vol. 313, 573-575, 1985)).

Sources of Thermal Hysteresis Proteins

Thermal hysteresis proteins (antifreeze proteins - AP) were found first in the body fluids of marine teleost fish which are hypoosomotic, have a blood serum freezing point of -0.7°C, but inhabit the polar ice-laden waters (Scholander et al. J. Cell Comp. Physiol., Vol. 49, 5-24, 1957). The first AP's were found by DeVries (Doctoral Thesis, Stanford, 1968) in Antarctic nototheniid fish. Two types of thermal hysteresis proteins have been isolated from polar and temperate fish, glycopeptide (AFGP) and peptides (AFP). In studies of fishes with two exceptions, the thermal hysteresis proteins are glycopeptides.

This type of thermal hysteresis protein, the antifreeze glycopeptides (glycoproteins) are present in eight distinct molecular weight classes ranging from - 2,500 to 34,000 daltons. They generally consist of a peptide backbone made-up of repeats of the tripeptide alanyl-alanyl-threonyl (the small glycopeptide may replace some alanines with proline beginning at position 7, with the disaccharide sugar beta-D-galactopyanosyl-(1→3)-2-acetamide-2-deoxy-alpha-D galactopyranose attached via a glycoside linkage to the hydroxyl side chain of each threonine (A. DeVries, Science, Vol. 172, 1152-1155, 1971).

These polypeptide or glycopolypeptides are obtainable from body fluids of fish. Preferably, the AFP's are obtained from the serum or body fluids of Arctic, Antarctic, North Temperate or South Temperate fish. More preferably, the serum and fluids of Arctic or Antarctic fish are used, eg see Table 1 below.

## TABLE 1

### MOLECULAR WEIGHTS OF THERMAL HYSTERESIS PROTEINS (AFGP)

A.    Thermal hysteresis proteins (AFGP) isolated from the Antarctic nototheniidae fish; <u>Pagothenia</u> <u>(Trematomus)</u> <u>borchgrevinki</u>, are:

| Glycopeptide Fraction No. | Molecular Weight (Approx.) |
|---|---|
| 1 | 33,700 |
| 2 | 28,800 |
| 3 | 21,500 |
| 4 | 17,000 |
| 5 | 10,500 |
| 6 | 7,900 |
| 7 | 3,500 |
| 8 | 2,600 |

Glycopeptide from <u>Dissostichus</u> <u>mawsoni</u> of structure

```
Ala-Ala-Thr-Ala-Ala-Thr-Ala-Ala-
         |             |
        NAGA          NAGA
         |             |
        GAL           GAL

Thr-Ala-Ala-Thr-Ala-Ala-Thr-Ala-Ala-
 |             |             |
NAGA          NAGA          NAGA
 |             |             |
GAL           GAL           GAL
```

The molecular weights of the fractions are essentially identical to those of Table 1 above.

Thermal hysteresis proteins isolated from the blood of Antarctic nototheniid fish exist in at least 8 sizes depending on the number of repeats of the basic glycotripeptide unit, see Table 1. The molecular weight ranges between 2,600 and 33,700 dalton (DeVries et al. (1970)). These AFGP's make up 3-4% of the blood of the fish and along with the sodium chloride, they lower the fish's freezing points below that of seawater.

The AFGPs inhibit the growth of ice crystals by adsorption to the ice crystal (Raymond et al. (1977), DeVries (1984)). Adsorption occurs on specific faces of the ice crystal (primary prism faces (1010)) resulting in inhibition of ice growth on these faces (DeVries, 1984), Consequently, in solutions of AFGPs, ice crystals grow predominantly on the basal plane (parallel to the c-axis), to which the AFGPs do not absorb, and take the form of very small, needle-like ice crystals (Raymond et al., (1977); Devries, 1988)). Peptide antifreezes can be found in several North Temperate, Arctic or Antarctic fish. The peptides vary in size and composition.

These polypeptide are essentially different lengths of the repeating tripeptide -alanine-alanine-threonine- where substantially each threonine is joined by a glycosidic linkage to the disaccharide B-D-galacto-pyransoyl-(1→3)- 2-acetamido-2-deoxy-alpha-galactopyranose.

The small glycopeptide may also have a small amount of proline located at positions 7, 10 and 13, but are otherwise structurally the same as the large glycopeptide.

Generally the higher the molecular weight, the more effective is the AFGP in promoting ice crystal growth along the c-axis, for example, fractions 1-5 above individually or as a mixture as obtained by purification. Fractions 6, 7 and 8, having a lower molecular weight, individually or as a group are apparently less effective in preservation.

The thermal hysteresis proteins (AFGP) are essentially similar in all the Antarctic Nototheniidae fish including (Pagothenia borchgrevinki, Trematomis Nicolia, Dissostichus Mawsoni (J.T. Eastman, and A.L.DeVries, A.L. Scientific American, Vol. 254, 106-114 (1986)). The same eight glycopeptides have also been isolated from northern hemisphere gadid, the rock cod (Gadus ogac) and in some other northern cods belonging to the family Gadidae (DeVries, A.L. Comp. Biochem. Physiol., Vol. 9OB, No. 3, pp. 611-621 (1988). All the AFGP's isolated so far are similar in structure with relatively small changes such as the position occupied by proline in AFGP 8 in northern species, or difference in size in northern cod, but all have essentially the same composition (A.L. DeVries, (1984) Phil. Trans. R. Socl. Lond., Vol. B 304, 575-588).

The other kind of thermal hysteresis proteins found in fish are polypeptides. While the AFGPs are in general polymers of a glycotripeptide unit Ala-Ala-Thr with disaccharide linked to the Thr side chain, the peptides are quite diverse structurally and vary in size and composition.

The thermal hysteresis protein from the winter flounder, Pseudopleuronectes americanus, although having a specific activity similar to that of the larger molecular weight glycoproteins, lacks sugars entirely and instead has high percentages of hydrophilic amino acids (especially threonine and Asp) while still retaining a large amount (about 60 mol %) of alanine. The flounder protein primary structure has clusters of hydrophilic amino acids separated by sequences of alanine, (Duman and DeVries (1976) Comp. Biochem. Physiol, Vol. 533, 375-380).

Peptides from Winter Flounder

Asp-Thr-Ala-Ser-Asp-
Ala-Ala-Ala-Ala-Ala-Ala-Leu-Thr-Ala-Ala-Asp-
Ala-Ala-Ala-Ala-Ala-Ala-Leu-Thr-Ala-Ala-Asp-
Ala-Ala-Ala-Ala-Ala-Ala-Ala-Thr-Ala-Ala.

Peptides from the Antarctic eel pout Rhigophila dearborni

| Peptide No. | Molecular Weight |
|---|---|
| 1,2,3 (three components) | 6,900 |

Asn-Lys-Ser-Val-Val-Ala-Asn-Gln-Leu-Ile-Pro-Ile-Asn-Thr-Ala-Leu-Thr-Leu-Ile-Met-Lys-Ala-Glu-Val-Val-Thr-Pro-Met-Gly-Ile-Pro-Ala-Glu-Asp-Ile-Pro-Arg-Ile-Ile-Gly-Met-Gln-Val-Asn-Arg-Ala-Val-Pro-Leu-Gly-Thr-Tyr-Leu-Met-Pro-Asp-Met-Val-Lys-Asn-Tyr-Glu-.

Other fish that produce thermal hysteresis proteins are listed in A.L. DeVries, Phil. Froms, R. Soc. London, Vol. 304, 575-588 (1984) such as the Alaskan plaice, Atlantic sculpins, Grubby Sculpin (Yang, D.S.C. Nature, Vol. 323, 232-237, 1988) and the Antarctic Eelpout (Rhigophila dearborni). Recent reviews of the thermal hysteresis proteins in fishes can be also found in (Feeney and Burchan (1986), Ann.Rev. Biophys, Biophys. Chem., Vol 15, 53-78,) and (Davies et al. (1988) Canadian J. Zool, Vol. 66, 2611-2617).

V.S. Ananthanarayanan, Life Chemistry Reports, Vol. 7, pp. 1-32 (1989), also describes sources of thermal hysteresis protein, particularly Type I, II and III.

These AFPs, AFGP's (or fractions and mixtures of fractions thereof) and others are available upon request from Dr. Arthur DeVries, Department of Physiology, Burrill Hall, 407 S. Goodwin, University of Illinois, Urbana, IL 61801.

These thermal hysteresis proteins or peptides of the present invention may also be produced by synthetic means. These means include the use of a peptide synthesizer available commercially in the art as Model 430A, Applied Biosystems, Inc., Foster City, California. The operation manuals for this peptide synthesizer are useful. The synthesis procedures of J.J. Nestor, et al., U.S. Patent 4,318,905, and R.B. Merrifield, U.S. Patent 3,531,258 are specifically incorporated herein by reference and are adapted for the preparation of the Ala-Ala-Thr and Ala-Ala-Ala compounds described above. Once the peptide is prepared, the threonine residues are optionally bonded to the disaccharide by conventional methods.

Hence, in summary, the thermal hysteresis protein used in the method of the invention suitably comprises a peptide having multiple regions of alanine-alanine threonine or alanine-alanine-alanine, or the glycopeptide having multiple regions of alanine-alanine-threonine, wherein covalently attached to substantially all of the threonine residues is the disaccharide, β-D-galactopyranosyl-(1-3)-2-acetamide-2-deoxy-α-D-galactopyranose. The molecular weight of the polypeptide or glycopeptide is between about 2,000 and 50,000 daltons. Where the thermal hysteresis protein is a glycopeptide the molecular weight may be between 2,200 and 40,000 daltons.

The thermal hysteresis protein used in the method of the present invention are independently selected from the protein themselves, or glycoprotein, or the protein or glycoprotein covalently bonded to a carrier such as biologically compatible antibody, gelatin, biocompatible polymer, peptide, sugar, or carbohydrate. Mixtures of these thermal hysteresis proteins are contemplated for use in the method of the present invention. Covalent bonding of a protein to a carrier by methods known to those of ordinary skill in the art are, for example, found in K. Rubenstein, et al., U.S. Patent 3,817,837, or M. Goodman et al., U.S. Patent 4,837,305.

## GENERAL

In the method of the present invention using aqueous composition of the said thermal hysteresis proteins, the process of ice freezing in tissue is changed, and the structural damage to the tissues reduced or eliminated through modification of the pattern of ice crystal growth. This advance is accomplished by modifying the pattern of ice crystal growth in tissue using compositions of thermal hysteresis proteins, e.g., peptides or glycopeptides, obtainable from Arctic or Antarctic fish. The effect of the antifreeze proteins on the freezing pattern in aqueous solutions is documented extensively as cited above. The different thermal hysteresis proteins obtainable from different fish sources adsorb to different crystal faces, however, all intifreeze protein inhibit ice crystal growth parallel to the a-axes, thermodynamically preferred direction of growth. Freezing in the presence of any kind of thermal hysteresis protein invariably leads to ice crystals forming in the direction of the c-axis. The ice crystals grow in the form of spicules (spikelike structures). These small micron and submicron scale spicular structures are stable and entrap between them the solutes that are rejected during formation of ice.

## SOLUTION PREPARATION

Compositions comprising an aqueous solution thermal hysteresis protein (AFP or APGP) are prepared by any number of methods. Water (usually sterile) is contacted with AFP of AFGP and mixed to produce a solution of between about 0.1 and 100 mg/ml solute in water. Usually the thermal hysteresis protein saturates in water in concentrations greater than about 100 mg/ml. Preferably, a solution of between about 1 and 60 mg AP/ml is produced, especically between about 20 to 40 mg/ml. The aqueous phase may also contain salts, sugars, ions nutrients (e.g. Krebs solution) and mixtures thereof in concentrations known in the art to be useful for preserving biological agents. The aqueous phase may also contain other materials, e.g. glycerol, etc., which are useful in the preservation of tissue, cell membrane, etc.

For use in organ transplantation and the like, sterile conditions and solutions must be used. The solutions may be prepared using sterile materials and sterile conditions. Also the solutions may be sterilized by methods known in the art, e.g. brief exposure to cobalt-60 radiation.

## TISSUE PRESERVATION

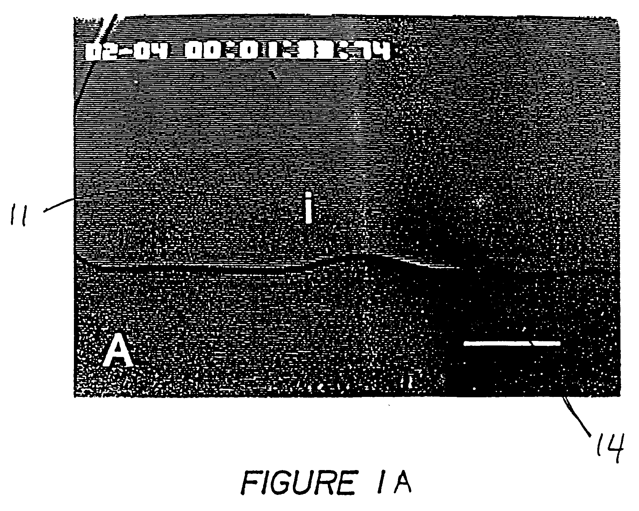

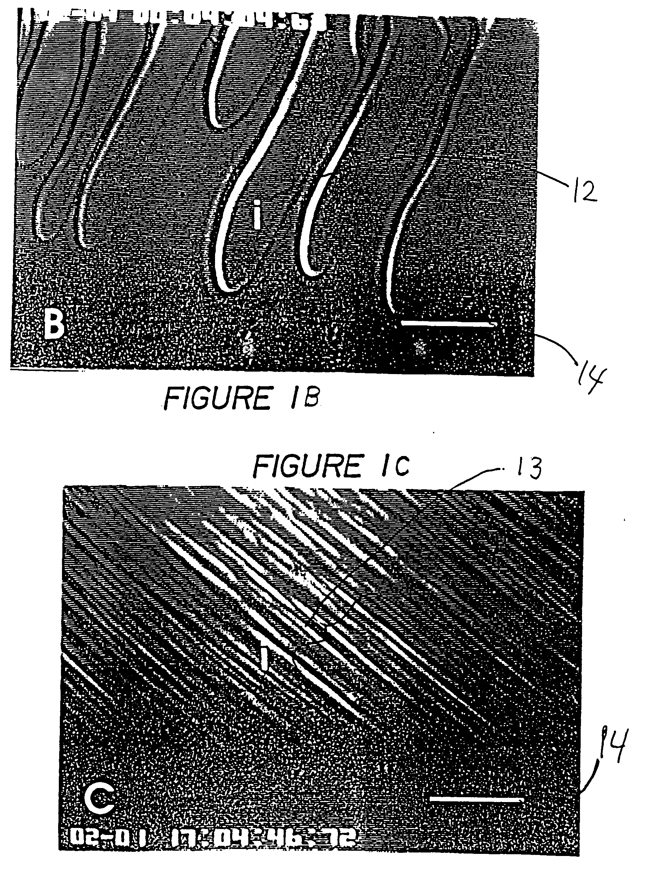

To illustrate the effect of thermal hysteresis proteins on the ice crystal structure, experimental results of the present invention are presented from earlier research in which the freezing pattern in a physiological saline solution is compared to the freezing pattern in a physiological solution with the addition of between about 1-100 mg/ml preferably about 40 mg/ml of thermal hysteresis protein from Antarctic nototheniindae fish (Table 1). In this comparison, samples are frozen under controlled thermal conditions on a directional solidification stage. The directional solidification stage, described in greater detail in U.S. Patent 4,531,373 is an apparatus capable of freezing solutions or tissue samples with uniform cooling rates, between predetermined temperatures. The apparatus is used in conjunction with a light microscope to produce results shown in Figures 1A, 1B and 1C which demonstrate the spicular growth in the presence of antifreeze glycoproteins.

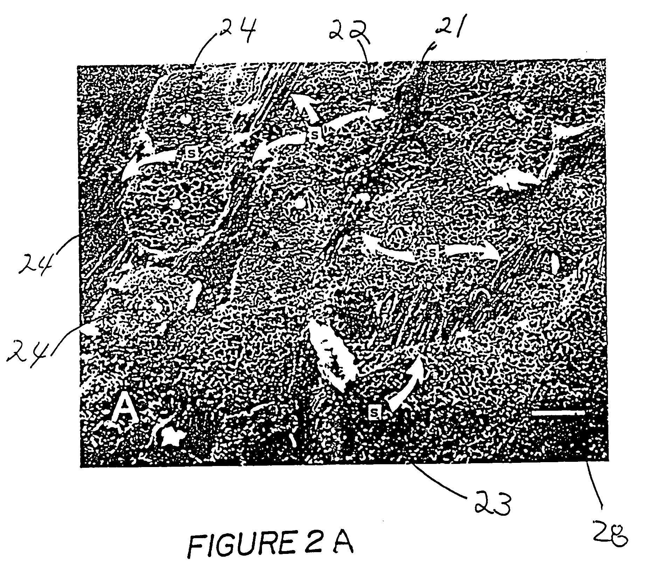

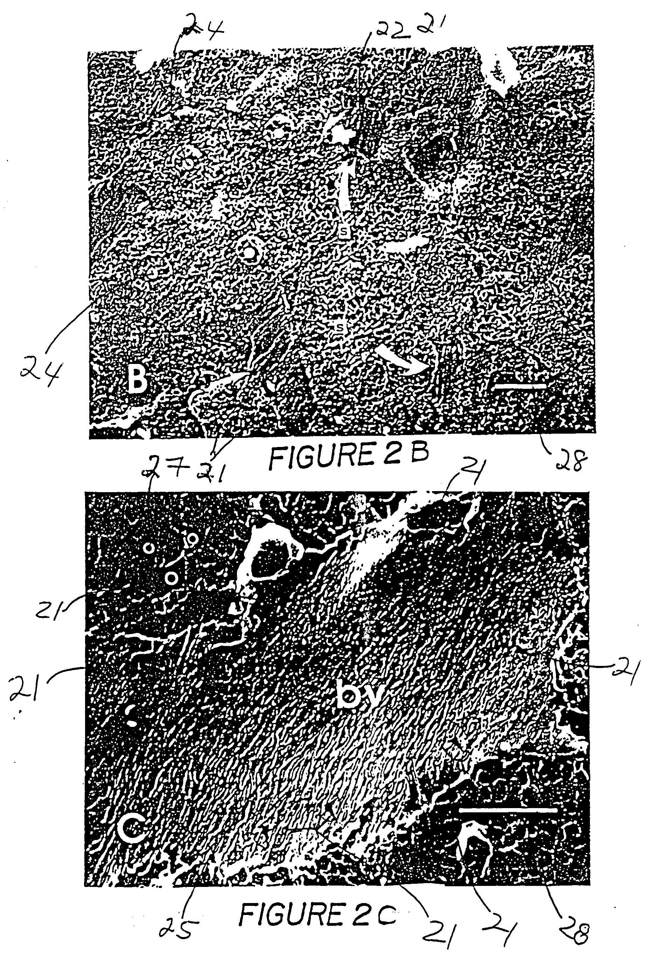

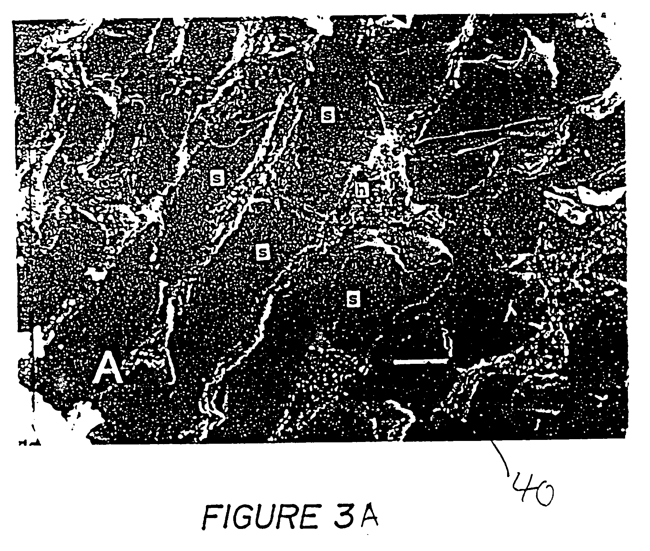

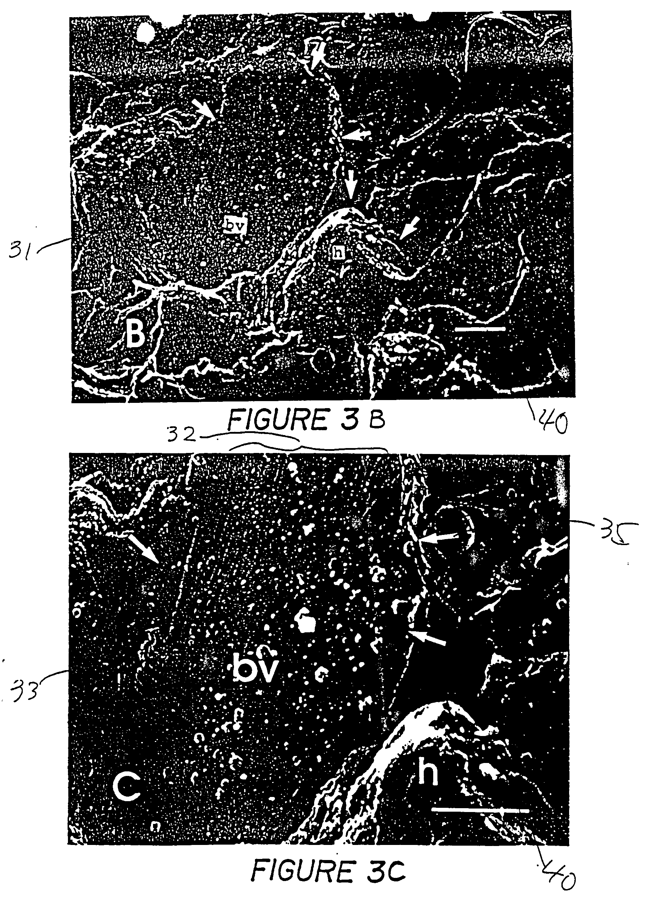

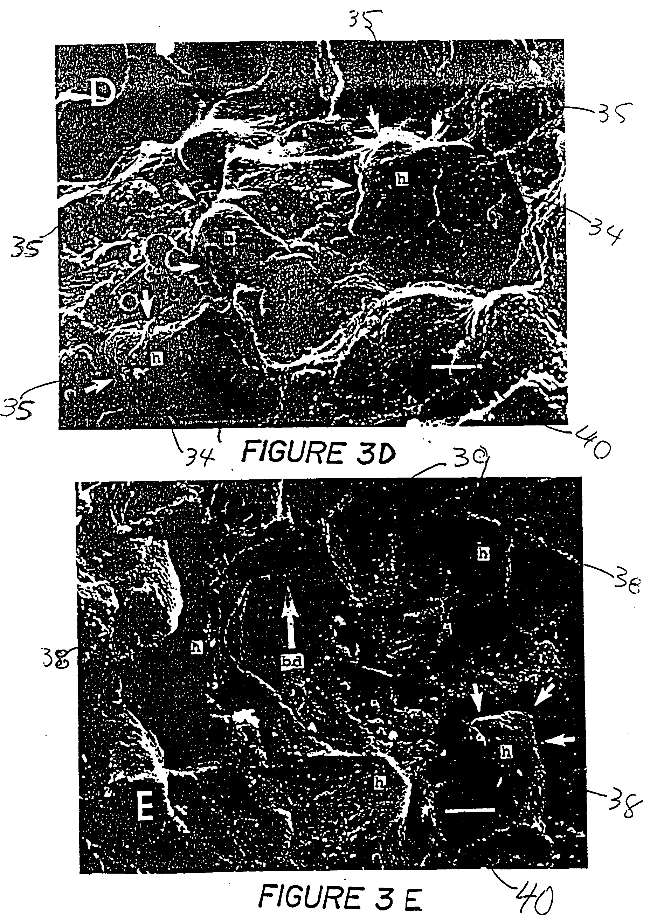

One embodiment of the present invention is to perfuse solutions containing thermal hysteresis proteins through the vasculature of an organ. Upon freezing, the ice crystals that form will be small, spicular and will entrap the solutes present. Consequently the cells will not be exposed to high saline concentrations, and the damaging expansion of the blood vessels will be eliminated. This effect will be demonstrated with detailed experimental results using the directional stage and scanning electron microscope in Figures 2A, 2B, 2C and 3A and 3B, 3C, 3D and 3E.

## VITRIFICATION

It was formerly observed that cells, tissue or organs may not survive freezing with rapid cooling or "apparent vitrification". The expression "apparent vitrification" is used here to describe the observation that, at times, a solution is considered to be vitrified if it remains transparent after rapid cooling to cryogenic temperatures. However, the property of transparency is only an indication that the ice crystals are either too small or too few to reflect light and therefore, the vitrification is only apparent. In one aspect of the invention, it was expected that mammalian cells, tissues or organs preserved by techniques in which the solution containing the cells (or organs, tissue) is frozen by rapid cooling or apparent vitrification may be damaged by the preferential formation of very small ice crystals on the cell membrane, which may serve as a nucleation site. The thermal hysteresis proteins inhibit the growth of ice crystals and significantly reduce the size of the crystals formed by generating spicular ice structures. Therefore, these biologically compatible thermal hysteresis proteins probably enhance the effectiveness of cryopreservation by preventing the formation of ice crystals on the cell membrane or by reducing the size of these ice crystals.

The effectiveness of the thermal hysteresis proteins (in vitrification) was evaluated on the cryopreservation of immature pig oocytes, two-cell stage pig embryos and mouse embryos, at the two-cell stage frozen by rapid cooling and "apparent vitrification." Pig oocytes and pig embryos at the two-cell stage were chosen because they present a very challenging model for which no successful cryopreservation has been heretofore achieved. In fact, pig oocytes and early-stage pig embryos usually cannot survive exposure to temperatures as high as 10°C for even brief time periods.

The probability of ice crystal nucleation during cooling is an inverse function of viscosity and temperature and a direct function of volume (D. Turnbull, 1969). In cryopreservation by rapid cooling, attempts are made to reduce the probability for nucleation by increasing the solution viscosity and by reducing the phase transition temperature through an increase in the concentration of various cryoprotectants. However, higher concentrations of cryoprotectants have a damaging effect on biological materials and, therefore, a proper balance must be found between a concentration that is sufficiently high to suppress nucleation and sufficiently low to avoid damaging the fragile cells.

These experiments were performed by exposing droplets of different size and composition to a variety of cooling rates on a special experimental system developed by B. Rubinsky, U.S. Patent 4,531,373. Rapid cooling, as well as rapid warming of samples, was performed using a Leitz Diaplan microscope to which a special directional stage was attached (A. Arav et al., 1990; B. Rubinsky, 1985; B. Rubinsky et al., 1985). The stage allows accurate control of cooling and warming rates between predetermined temperatures particularly as it is applied to vitrification and freezing by rapid cooling. A video camera was used in conjunction with the microscope to evaluate the morphology of the cells and the physical state of the solution.

An "apparent vitrification solution", (AVS) was useful which contains 17.5% propylene glycol, (Fluka Chemicals, Switzerland), 2.5% glycerol (BDH Analar, England), 20% FCS (Fetal Calf Serum) (Gibco, Scotland) and 0.05 M sucrose in PBS (Dulbecco's phosphate buffered saline supplemented with 0.4 m/v BSA (Bovine Serum Albumin), 0.34 mM pyruvate, 5.5 mM glucose and 70 μg/ml kanamycin).

This solution is physiologically compatible with mouse and pig embryos and with pig oocytes. When 0.1 μl droplets of the AVS solution were cooled at the rate of 1,700° C/min (the highest rate possible with the directional solidification stage) to a temperature of -130° C (a temperature lower than the glass formation temperature for this solution) no ice crystals were observed through the microscope at 340x magnification. To illustrate the effect of volume and solute concentration, ice crystals were observed with all droplets of the AVS solution larger than 0.5 μl, and with all 0.1 μl droplets containing 12.5% propylene glycol and 2.5% glycerol when cooled at 1,700° C/min. No apparent devitrification, (that is, the formation of ice crystals was observed with droplets of the AVS solution larger than 0.5 μl, and with all 0.1 μl droplets containing 12.5% propylene glycol and 2.5% glycerol when cooled at 1,700° C/min.) No apparent devitrification (i.e., ice crystal formation) was observed when the samples were held at -130° C. However, devitrification was observed in some samples during warming to room temperatures even when the rate was as high as 1,700° C/min. The addition of thermal hysteresis proteins (AFGP's or AFP's) to the AVS solution did not preclude the seldom and random occurrence of devitrification after "apparent vitrification". The AVS was the basic solution used in the experiment are reported to evaluate the effects of freezing with rapid cooling for used droplets larger than 0.5ml and for "apparent vitrification" droplets of 0.1ml. In the vitrification studies, only results from solutions that did not undergo devitrification were evaluated.

Evaluation of Cryoprotective Properties of Thermal Hysteresis Proteins in Oocytes and Embryos

To evaluate the cryoprotective properties of the thermal hysteresis proteins, immature pig oocytes, two-cell stage pig embryos and two-cell stage mouse embryos were introduced into either 0.1 ml droplets for vitrification, or droplets larger than 0.5 ml for freezing with rapid cooling of AVS with, and without, thermal hysteresis protein. These droplets were cooled on the directional stage under microscope observation at the rate of 1,700° C/min to -130° C. After 15 minutes at these temperatures, the samples were warmed at the rate of 1,700° C/min to room temperature. The survival of the embryos and oocytes was evaluated by in vitro culture followed by morphological and development analysis. Control experiments were performed by exposing embryos and oocytes to the different solutions in protocols identical to the rapid cooling experiments, but without cooling and warming, and evaluating their viability. The thermal hysteresis proteins used in this work were obtained from Antarctic fish belonging to the family Nototheniidae (Dissostichus Mawsoni) (Table 1). A physiological composition was used which consists of one part of fraction 1 to 5 (high molecular weights) and two parts of fraction 7 and 8 (lower molecular weights) as obtained form A. DeVries, University of Illinois. Fractions 1-5 are obtained as a mixture, and fraction 7-8 are obtained as a mixture. Experiments were performed with solution concentrations of 40 mg/ml glycopeptides. PBS is a standard buffered solution. This particular value was chosen because studies have shown that the depression of the freezing point of aqueous solutions of antifreeze glycoproteins is concentration-dependent and at these concentrations, it reaches saturation. A.L. DeVries, (1988).

After the cryoprotective properties of the thermal hysteresis proteins (AFGP's) were established, parametric studies were performed with two-cell stage mouse embryos to determine the effect of concentration on the survival of the embryos. This animal model was chosen for parametric experiments because it proved extremely sensitive to the effect of the glycopeptides. While no survival of embryos was achieved without the thermal hysteresis proteins (0%), very high survival of embryos was obtained with the thermal hysteresis protein (82.5%, in vitro development to the blastocyst stage). The experimental procedures for pig oocytes and pig embryos are found in Example 3 below, and for mouse embryos is found in Example 4.

The protocol to which the cells were exposed is the one described above in which the embryos and oocytes were introduced in various solutions with some of the embryos and oocytes exposed to rapid cooling while others which did not undergo cooling, kept as controls for the solution effect. The results are presented, for pig oocytes, as the ratio between the number of oocytes which reached the MI or MII stage after in vitro maturation, and the total number of oocytes exposed to the experimental protocol. For the pig embryos, the ratio between the number of embryos that reached the four-cell stage after in vitro development and the total number of embryos exposed to the experimental protocol was measured. For the mouse embryos, it is the ratio between the number of embryos that reached the blastocyst stage over the total number of embryos exposed to the experimental protocol was measured.

The experiments with pig oocytes, pig embryos, and mouse embryos exposed to the AVS solution, show that this solution does not have a damaging effect. However, when the embryos and the oocytes were cooled rapidly or vitrified to cryogenic temperatures in the AVS solution, not a single embryo or oocyte survived. These results demonstrate that the damage to these cells is a consequence of cooling and exposure to cryogenic temperatures. Microscopic examination revealed that a primary site of damage following rapid cooling in the AVS solution was the oolemma in the case of oocytes and the blastomer membrane for embryos which did not retain integrity as illustrated in the Figures, especially in Figs. 4B, 5A, and 6B. However, in the presence of the thermal hysteresis proteins the cells that were rapidly frozen or vitrified retained viability.

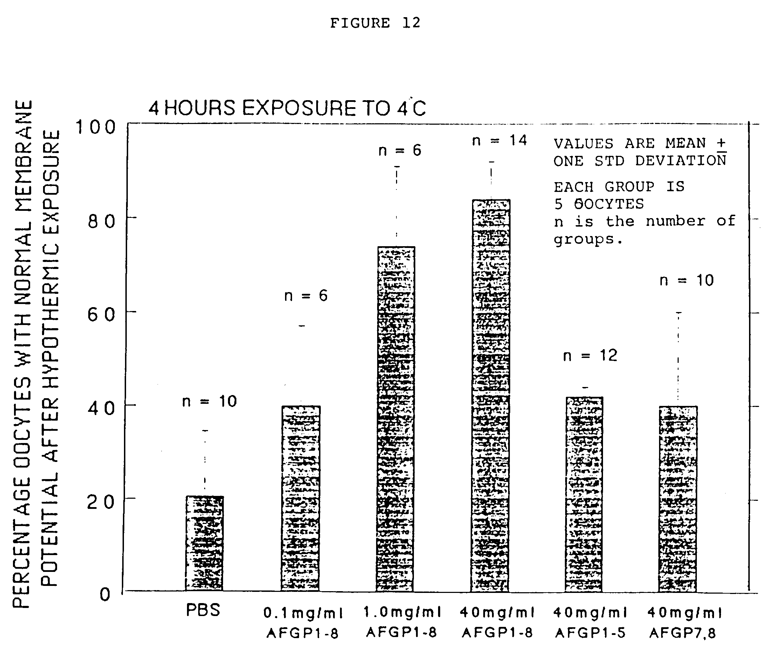

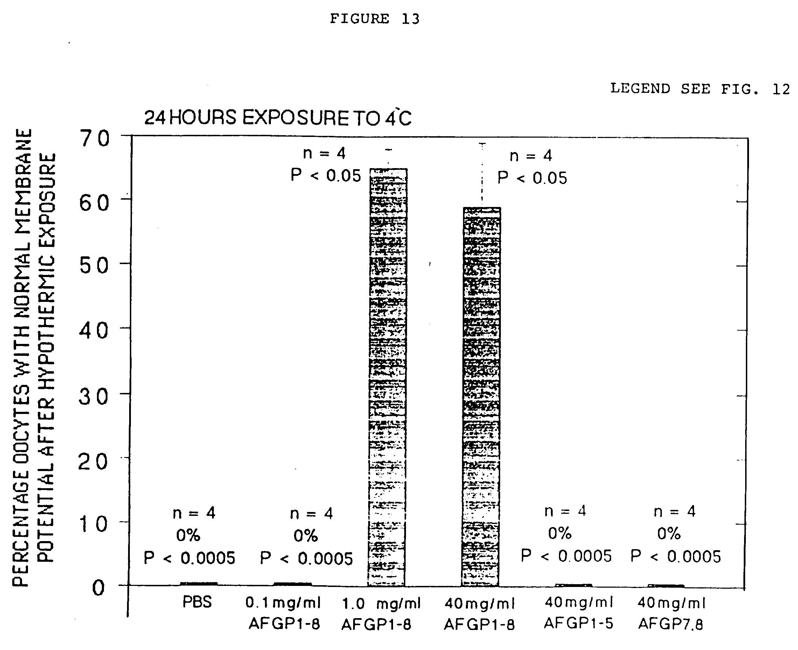

In Figures 12 and 13 are shown the membrane potential for oocytes at 4 and 24 hr at 4°C. The dramatic retained membrane potential viability at concentrations of 1-40 mg/ml of thermal hysteresis proteins is found in Figure 13. Figures 12 and 13 values are mean ± one standard deviation. Each exp. group consists of 5 oocytes and n represents the number of groups.

In particular, the cell membrane was protected by the glycopeptides.

UTILITY

It is apparent from the disclosure herein that the aqueous composition of thermal hysteresis protein is useful in cell preservation including membrane preservation, tissue preservation or organ preservation.

In general, the thermal hysteresis proteins have the property that they noncolligatively lower the apparent freezing point of aqueous solution resulting in a freezing temperature that is lower than the melting temperature. They also have the general property that they inhibit or restrict growth on different facets of ice crystals while allowing the growth along the c-axis.

EFFECT OF THERMAL HYSTERESIS PROTEINS ON THE CELL MEMBRANE

Initially, the effect of the thermal hysteresis proteins on modification of ice crystal growth focused on the use of this property in preservation of cells, tissue or organs at temperatures below freezing. However, in studies described above and experiments such as Examples 1 and 3 , in which the morphology of cell membranes was evaluated, it appeared consistently that the thermal hysteresis proteins provide complete protection to the morphology of the membrane and its structural integrity. Therefore, a procedure was developed to determine if the thermal hysteresis proteins protect by interacting directly with cell membranes, by contacting the protein directly to cell membranes.

Pig oocytes were chosen as the experimental model in this study because these oocytes are temperature sensitive and cannot survive exposure to hypothermic temperatures as high as 10°C, i.e., temperatures that are higher than the

## TABLE 2: EFFECT OF AFGP ON OOCYTE VIABILITY

| Sol'n | Time of Exposure (hr) | PBS | PBS + 0.1 mg/ml AFGP 1-8 | PBS + 1 mg/ml AFGP 1-8 | PBS + 40 mg/ml AFGP 1-8 | PBS + 40 mg/ml AFGP 1-5 | PBS + 40 mg/ml AFGP 7,8 |
|---|---|---|---|---|---|---|---|
| + | 4 | 6/48 (12.5%) | 7/25 (24%) | 19/27 (70%) | 54/70 (77%) | 12/43 (28%) | 11/47 (23%) |
| * | 4 | 9/48 (18.75%) | 11/29 (37%) | 20/27 (74%) | 59/70 (84%) | 21/43 (48%) | 18/47 (38%) |
| + | 24 | 0/17 (0%) | 0/14 (0%) | 6/14 (42%) | 7/17 (41%) | 0/14 (0%) | 0/13 (0%) |
| * | 24 | 0/17 (0%) | 0/14 (0%) | 9/14 (64%) | 9/17 (53%) | 0/14 1(0%) | 0/13 (0%) |

+ CRITERIA A = $\dfrac{\text{number of cells with electrical potential} > |u| - |v|}{\text{total number of cells}}$

* CRITERIA B = $\dfrac{\text{number of with electrical potential} > |u| - |2v|}{\text{total number of cells}}$

phase transition temperature. Therefore, an experiment was designed in which the effect of the thermal hysteresis proteins (AFGP's) on the cell was studied at temperatures higher than the phase transition temperature, but lower than the normal body temperatures. If a protective effect of the AFGP's is found, it is probably not directly related to the ability of the compound to modify ice crystal morphology or inhibit of ice crystal formation.

The oocytes were introduced in different solutions of standard buffer PBS solution with thermal hysteresis proteins (Fractions 1-5, Table 1) from fish of the family Nototheniidae. They were kept in a constant temperature environment for various periods of time and then the membrane potential was measured. The structural integrity was also determined by microscope evaluation.

To establish a criteria for an intact oolema, preliminary experiments were performed for each batch of oocytes in which the membrane potential of the fresh oocytes was measured at 22°C. The mean value of the electrical potential, u, and the standard deviation, v, were calculated for each batch. The mean and the standard deviation were measured in fresh oocytes in a buffer solution and in a buffer solution with 40 mg/ml antifreeze glycopeptides Fractions 1-8 (Table 1) as obtained from A. DeVries, supra.

Table 2 above summarizes the results derived from measuring the resting potential across the oolemma. Table 2 gives the ratio between the number of oocytes considered to have an intact oolema relative to the number of oocytes used for each experimental condition, (the number in the brackets is the ratio in percentage,) for different concentrations of the thermal hysteresis proteins (AFGP's) and different times at 40°C.

The comparison shows that the glycopeptides have little effect on the resting potential of each oocyte. To determine the integrity of the oolema, two statistical criteria were established, one less stringent than the other. The oolema in an oocyte was considered to be intact if the absolute value of the measured resting potential difference, was higher than the absolute value of either $|u-v|$ or $|u-2v|$.

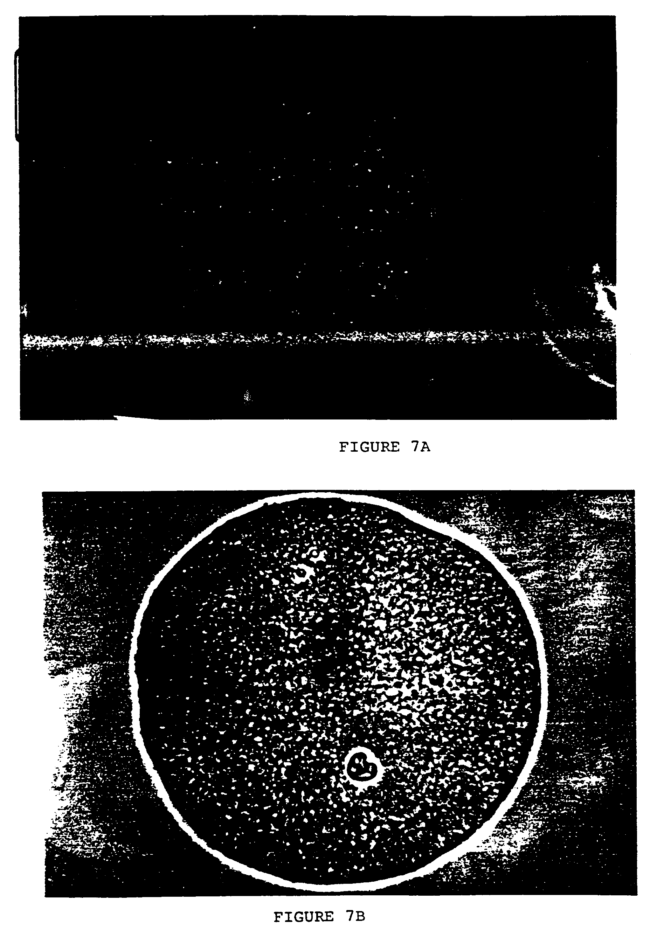

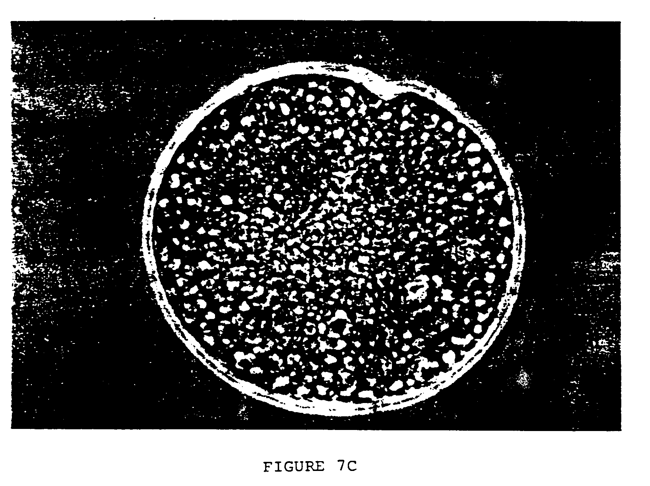

The results from evaluating the structural integrity of the oolema are consistent with the electrical potential measurement and are illustrated by Figures 7A, 7B, and 7C. The results clearly show that the membrane is preserved morphologically intact in the presence of the thermal hysteresis proteins. Furthermore, ion leakage that is probably the most prevalent cause of damage during hypothermia exposure is significantly inhibited in the presence of the thermal hysteresis proteins. This implies that the thermal hysteresis proteins have the ability to protect cell membranes at hypothermic temperatures and to block ion channels. The evidence of the use of this new discovery in hypothermic preservation of cells and organs are given below in Examples 5 and 6, respectively.

WHOLE ORGAN PRESERVATION

Cryopreservation of a whole organ, e.g. liver from a mammal, such as a rat, is described in Examples 2, 6 and 6A below. The organ is surgically removed, held in a preservation solution at 20-37°C, preferably 24°C. A major blood vessel is cannulated. The well-known Langendorf perfusion system (with a first bottle containing, for example, Krebs solution and antifreeze polypeptides in a 1mg/ml to 100 mg/ml) is used. See, for example, D.E. Pegg et al. (1986), Cryobiology, Vol. 23, pp. 150-160.

A second bottle of solution contains a physiologically compatible saline solution and appropriate quantities of glycerol, dimethyl sulfoxide, ethylene glycol, polyvinyl pyrrolidone, glucose etc. or mixtures of these substances which are known as protectants for cells of biological origin.

These two bottles of solutions are connected to a mixing valve having known adjustable flow rates (e.g. 0.1 to 10 ml/min, preferably about 5 ml/min) and a computer to accurately vary the flow rate and mixing of each bottle's contents immediately prior to perfusion. The perfusion using the solutions of bottles 1 and 2 is well known in the art as described by D.E. Pegg et al. (1988) above for kidney and G.N. Alink et al. (1976), Cryobiology, Vol. 13, pp. 295-304; (1977) Cryobiology, Vol. 14, pp. 409-417 and 399-408; and (1978) Cryobiology, Vol. 15, pp. 44-58, and K.E.F. Hobbs et al. (1969), Cryobiology, Vol. 6, pp. 239-245 for heart. The Krebs solution is perfused through the organ held at about 20-37°C at a rate of about 4 ml/min.

The mixing switch provides intermediate amounts of Krebs solution and glycerol solution in pulses of time lengths controlled by the computer, for example, 0.01 sec. to 0.1 sec. The two solutions mix in the delivery tube or in a special mixing chamber.

The thermal hysteresis protein/Krebs solution is initially adjusted so that at the end of the perfusion process a concentration of between 1mg/ml to 40 mg/ml is achieved in blood vessel space in the tissue. The majority of the thermal hysteresis proteins are found within the vascular space (bed) of the organ (not within the cells of the liver or the blood vessels). The thermal hysteresis proteins are usually too high in molecular weight to significantly penetrate the cell membrane. The organ, e.g. liver, is next placed in a cooling stage as described in U.S. Patent 4,531,373, and the temperature of the whole perfused organ is then cooled at a rate of 1°C per minute to -32°C or to -70° or until -150°C is reached. The organ is then cooled as needed using a liquid nitrogen to -196°C or in liquid helium to -269°C (4K) and held at this temperature for an indefinite time (e.g. 72 hr). The frozen organ is then carefully thawed by immersion in a cold or warm liquid, e.g. water or saline, at a rate of between about 0.1 to 10°C per min. (preferably about 1°C per min.) using known techniques up to 37°C maximum. Alternatively, carefully controlled microwave heating is used to thaw the perfused organ, e.g. liver. When the thawed organ (liver) reaches about 0°C, the nutrient solution of Krebs is perfused through the large cannulated blood vessel. When warmed to about 20 to 37°C, preferably 37°C, the thawed organ recovers not only cell function, but also organ function. Preserved tissue samples are taken as needed.

A systematic study of the effects of the thermal hysteresis proteins on rat liver cold-storage was done to compare control storage solutions and solutions containing the AFGP (see Example 6A). The results obtained are compared for three different storage periods, 6, 12 and 24 hr. The functional tests include the production of bile and enzymatic activities of lactic dehydrogenase (LDH). The Krebs solution is selected as the control solution. The reason for this selection is to separate the protective qualities of other storage solutions from the effects of the AFGP.

Useful mammalian organs include liver, kidney, heart, brain, lung, pancreas, spleen, ovary, stomach and the like. The organ of a mammal, such as a human being, is preferred.

PRESERVATION OF CELLS AND TISSUE AT TEMPERATURES BELOW FREEZING

The cryopreservation is demonstrated in cells, e.g. human ooytes, pig oocytes, embryos, human or leucocytes, platelets, e.g. pancreatic islets, hepatocyles, corneas, skin. Various cryoprotective agents such as glycerol, propylene glycol are introduced in the cell together with the thermal hysteresis proteins essentially as described in Examples 3 and 4. The different solutions of cryoprotective agents are chosen to either produce freezing or vitrification such as 5M propylene glycol. The cells or tissues are then cooled rapidly to either produce freezing or vitrification with cooling rates of e.g. 1750 °C/min or as high as required to temperatures of -130°C to -180°C, or to -269°C (4K) and held at that temperatures for an indefinite period. The cells or tissue are then carefully thawed. Cell function and tissue function is recovered.

PRESERVATION OF ORGANS BY RAPID FREEZING VITRIFICATION

The procedure is the same as described in the whole organ preservation section except that the concentration of the cryoprotectant is taken to a high level, such as 5M propyleneglycol, and the cooling rates are high enough, such as 1,750°C/min, to produce either rapid freezing or vitrification as desired as opposed to slow freezing in the earlier application. The use of thermal hysteresis proteins is essential for the successful preservation of organ tissue by vitrification.

HYPOTHERMIC PRESERVATION OF CELLS

The procedure of Example 5 is followed except that liver cells are first contacted with aqueous thermal hysteresis protein solution. These cells survive the cooling and are viable upon careful warming to physiological temperatures.

HYPOTMERMIC PRESERVATION OF ORGANS IN COLD STORAGE

The procedure of Example 5 is followed except that an organ, e.g., liver or heart is contacted with the aqueous thermal hysteresis solution. This organ survives the cooling and is viable upon careful warming to physiological temperatures.

HYPOTHERMIC PRESERVATION OF ORGAN BY CONTINUOUS PERFUSION

Example 6 is repeated except that the blood containing the thermal hysteresis protein is continuously perfused through the organ.

HYPOTHERMIC PRESERVATION OF CELLS, TISSUE, ORGANS

Example 5 is repeated with the exception that thermal hysteresis proteins are brought in contact with cells, tissue and organs where it is desired to protect them from hyperthermic damage.

PRESERVATION OF CELL MEMBRANES

Cell membranes are brought into contact with physiologically capatible solutions with thermal hysteresis proteins.

ATTACHMENT THROUGH ANTIFREEZE PROTEINS

Various macromolecules are artificially attached to thermal hysteresis protein and then introduced to a cell suspension, tissue or organs. The thermal hysteresis protein attaches then to cell membranes and thereby bring the macromolecules in the vicinity of the cell membrane.

DETAILED DESCRIPTION OF THE FIGURES

The following is a detailed description of the Figures:

Figures 1A, 1B and 1C

Figure 1A shows the frozen region 11 (i) and the planar solid-liquid interface in a physiological saline solution at the onset of the normal freezing process.

Figure 1B shows the final dendritic, finger-like structure of ice 12 (i), during freezing of a physiological saline solution. Figure 1C shows spicular structure of ice crystals 13 (i), during freezing of a physiological saline solution with 40 mg/ml AFPs. The scale bar 14 shown in Figures 1A, 1B and 1C is 50 micrometer.

Figures 1A, 1B and 1C show the ice crystal morphology in aqueous solutions frozen with a cooling rate of 4° C/min on the directional solidification stage. Figures 1A and 1B show a well-known sequence of events during freezing of saline solutions. In saline solutions, ice forms and grows on the prism plane of the ice crystal, forming wide macroscopically smooth surfaces (Fig. 1A). During freezing, the ice rejects the solute which accumulates at the ice-water interface. The increased concentration of salts causes a colligative decrease in the change of phase temperature on the interface and lead, through the well-known phenomenon of constitutive supercooling instability, to the transformation of the ice crystal morphology from a planar structure to a dendritic one as shown in Figure 1B. However, Figure 1C shows that the ice crystal growth pattern in the presence of thermal hysteresis proteins (40mg/ml) is very different. Figure 1C shows spicular (spike-like) ice crystals much smaller in dimensions from the dendritic ice crystals seen in Figure 1B. The thermal hysteresis proteins ice structure is spicular from the onset of the freezing process. Using polarized light, it is shown that the spicular ice crystals grow in the direction of the c-axis. The small spicular ice crystals incorporate between them, the thermal hysteresis proteins and the other solutes in the solution. Figures 1A, 1B and 1C show that the incorporation of the solutes stabilizes the spicular ice growth along the c-axis. It is observed that in the presence of thermal hysteresis proteins the ice crystals are very small. The saline solution is incorporated between the submicron size ice spicules. Therefore, the salt solution does not concentrate significantly to produce a significant change in chemical potential. As a result, in organs water does not migrate from the surrounding cells dehydrating, expanding the blood vessels, and collapsing these cells.

Figures 2A, 2B and 2C

Figures 2A and 2B have black 21 and white arrows 22 which point toward longitudinally and transversely sectioned sinusoids 23 (s), which show spicular ice crystals. All the spicular ice crystals are oriented in the same general direction. Structurally intact hepatocytes surround the sinusoids. The round nucleus is evident in some of the cells, where it is marked with a white dot 24. Rounded ice crystals are observed in all the cells.

Figure 2C shows higher magnification of the spicular ice structures in a large blood vessel 25 (bv). The margin of the blood vessel is shown with black arrows 26. Typical ice crystals in adjacent cells are marked with white circles 27. The scale bar 28 for Figures 2A, 2B and 2C is 10 micrometer.

Figures 3A, 3B, 3C, 3D and 3E

Figure 3A shows normal liver tissue frozen with a cooling rate of 4°C/min on a directional solidification stage, as described hereinabove. Continuous, smooth ice crystals are seen inside expanded sinusoids, (s). The adjacent hepatocytes (h) are dehydrated.

Figures 3B and 3C shows liver perfused with thermal hysteresis proteins (40 mg/ml), which are similar to those in Table 1 and frozen with a cooling rate of 4°C/min on a directional solidification stage. The cross-section through a large blood vessel 31 (bv) shows spicular ice crystals 31 confined within the blood vessel 33. (The surface of the blood vessel is marred by debris formed during the fracture.) The outline of box-like hepatocytes 34 (h) fractured along the cell membrane is marked by arrows 35. The dimensions and the shape of the hepatocytes are typical to that of normal hepatocytes.

Figure 3D shows liver tissue perfused with thermal hysteresis proteins and frozen with a cooling rate of 4°C/min on a directional solidification stage. The fracture is along the cell membrane, with cells removed in a staggered form leaving behind stair-like arranged hepatocytes 36 (h). The outline of box-like, normal size hepatocytes (h) is shown by the black arrows 37.

Figure 3E shows normal liver tissue frozen with a cooling rate of approximately 4000°C/min, and fractured along the cell membrane. The cells are removed in a staggered form leaving behind stair-like arranged, normal size, box-like hepatocytes 38 (h), shown by arrows. The bile duct 39 (od) (cannaculus) has been preserved intact. The scale bar 40 shown Figure 3A to 3E is 10 micrometer.

Figures 4A, 4B and 4C

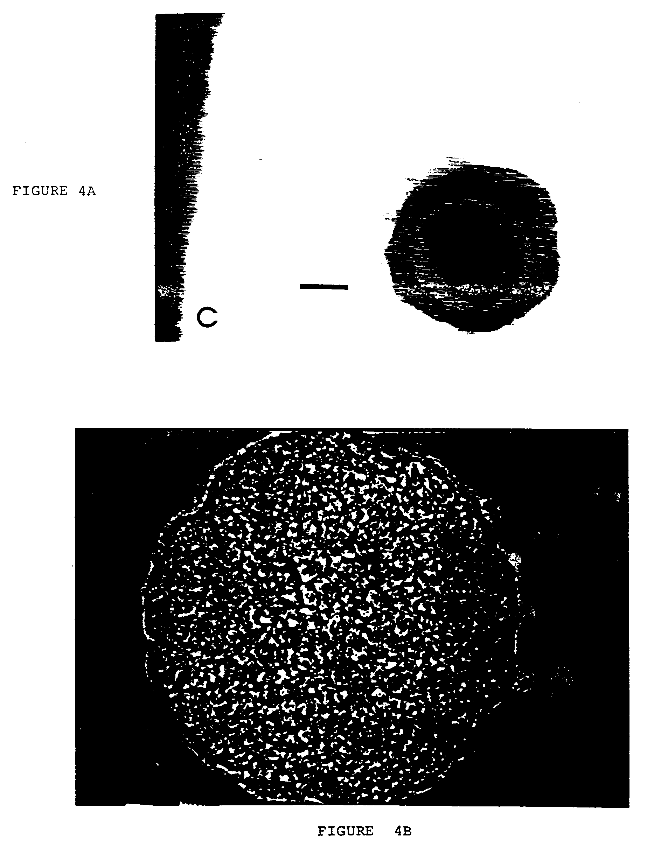

Figure 4 includes photographs concerned with the cryopreservation of immature pig oocytes.

Fig. 4A shows one pig oocyte in a transparent droplet during cooling to -130° C. The dark circular rim of the droplet is shown.

Fig. 4B shows a pig oocyte after rapid cooling to - 130° C in the AVS solution following 44 hours in vitro culture and stained. The cytoplasm is completely degenerated and the oolemma is not intact.

Fig. 4B illustrates the appearance of an oocyte that was not considered viable after rapid cooling in the AVS solution. The photograph shows an oocyte in which the membrane (oolemma) is not intact and no nuclear details are visible.

Fig. 4C shows an oocyte that survived rapid cooling to cryogenic temperatures in an AVS solution with thermal hysteresis proteins (AFGP) and consequently underwent in culture nuclear maturation to the MII stage. It must be emphasized that this is the first time any method has been developed under any conditions in which pig oocytes survive and develop in vitro after exposure to cryogenic temperatures.

Fig. 4C shows the appearance of an oocyte that reached the MII stage after rapid cooling to -130° C. The nuclear development stage is evident.

Fig. 4D shows a pig oocyte after rapid cooling to - 130° C in the AVS solution with thermal hysteresis proteins in Table 1 (40g/ml showing a normal morphology but no nuclear maturation (g.v. stage) after 44 hours of incubation: intact oolemma, intact g.v. membrane, normal cytoplasm morphology (the bottom of the photograph shows some cummulus cells). The scale bar for Fig. 4 is 50 $\mu$m.

Fig. 4D shows an oocyte cooled rapidly in the AVS solution with 40 mg/ml thermal hysteresis protein. This oocyte was not considered viable because it did not undergo nuclear maturation (remained at the g.v. stage). Nevertheless, it is noted that the cells show a normal morphology with an intact oolemma and an intact g.v. membrane. The intact appearance of the oolemma in Fig. 4D is typical to all oocytes cooled in the presence of the thermal hysteresis protein solution.

This set of experiments clearly demonstrates that the thermal hysteresis protein has a cryoprotective effect, which is associated with retaining the integrity of the cell membrane when exposed to severe temperature conditions.

Figures 5A and 5B

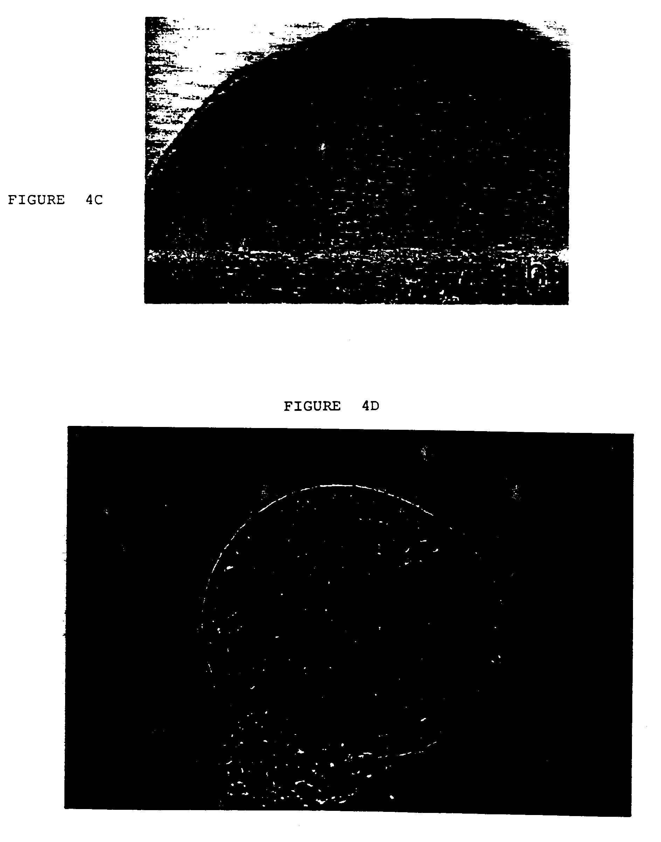

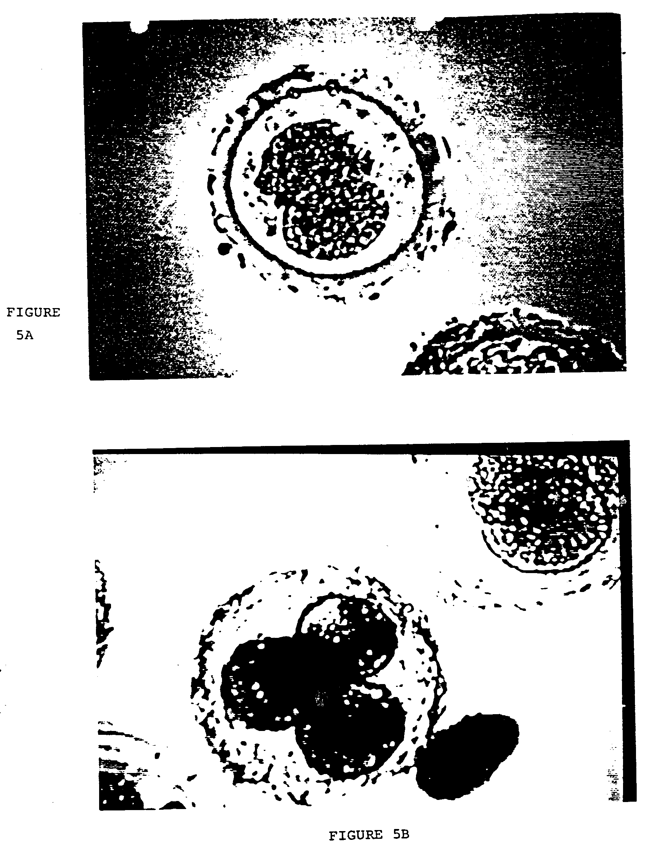

Fig 5 includes photographs concerned with cryopreservation of pig embryos at the two cell stage. The scale bar for Fig. 5 is 50 $\mu$m.

Fig 5A shows a two cell pig embryo after raped cooling to -130° C in the AVS solution and 24 hours in incubation. The complete disintegration of the blastomere membrane is evident.

Fig 5B shows a normal four-cell stage pig embryo developed from a two-cell stage embryo after rapid cooling to - 130°C in the AVS solution with thermal hysteresis proteins. The upper right-hand side of the photograph shows an embryo that remained at the two-cell stage. The clear integrity of the membrane is evident even in embryos that failed to develop. The scale bar for Fig. 5 is 50 $\mu$m.

This is the first report of a method for successful cryopreservation of pig embryos at the two-cell state.

Figure 6A, 6B and 6C

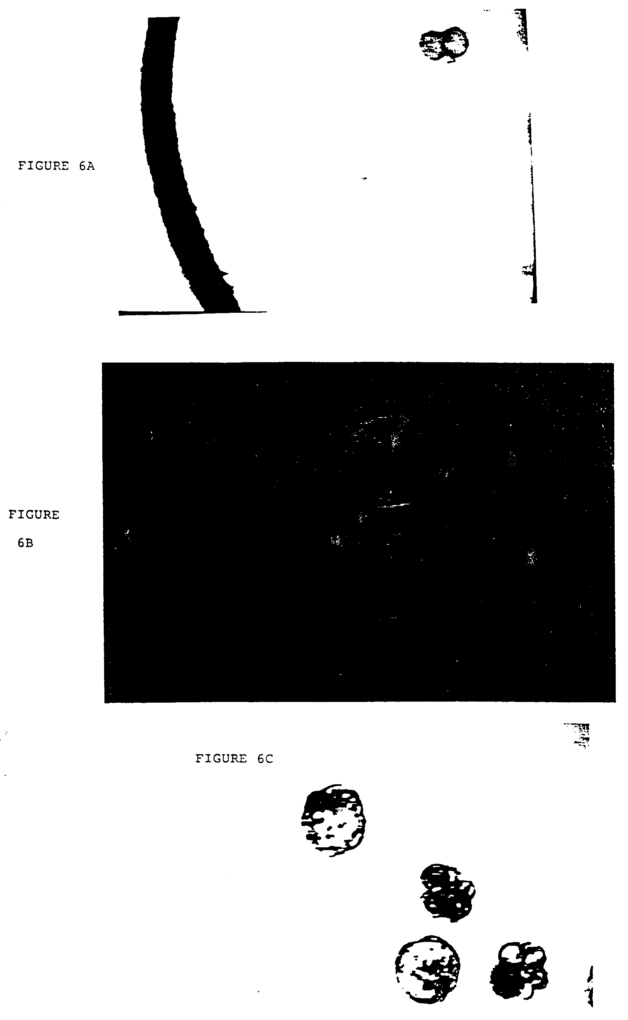

Fig. 6 includes photographs concerned with cryopreservation of mouse embryos at the two-cell stage. The scale bar for Fig. 6 is 50 $\mu$m.

Fig. 6A shows one mouse embryo in a transparent droplet during cooling to -130° C in the AVS solution after 72 hours incubation.

Fig. 6B shows one of the embryos remained at the two-cell stage with shrunken blastomere which implies membrane damage. In the second embryo, the blastomere membranes have disintegrated completely;

Fig. 6B illustrates the appearance of mouse embryos that did not survive rapid cooling in the AVS solution. The blastomere in one of the embryos in Fig. 6B are shrunk which implies loss of membrane integrity. The membrane disintegration is nearly complete in the blastomere of the other embryo shown in the photograph. The survival of rapidly cooled oocytes and embryos did not improve when 50 mg/ml AP were added to the basic AVS solution. The cell membrane integrity did not improve either and, in fact, there was absolutely no difference in survival or morphology between embryos and oocytes cooled in the AVS solution or the AVS solution with AP.

Fig. 6C shows the typical appearance of a blastocyst following in vitro culture of a two-cell stage mouse embryo cooled to -130° C in the presence of the thermal hysteresis protein.

The typical appearance of normal mouse blastocysts following cooling in the AVS solution with 40 mg/ml AFGP is shown in Fig. 6C. The high rate of survival of mouse embryos in the presence of thermal hysteresis proteins, 82.5% compared to no survival, 0% without thermal hysteresis proteins, provides a clear indication of the cryoprotective properties of the thermal hysteresis proteins.

As significant as these results are on survival, equally important are the microscopic observations showing that the integrity of the oolemma in the pig oocytes and of the blastomere membrane in the pig embryos is retained when the cooling occurs in the presence of thermal hysteresis proteins. The microscopic evidence shows that the membrane was intact in 35 of the 45 pig oocytes (82.2%) and in the blastomere of 23 of the 23 (100%) pig embryos when the oocytes and embryos were cooled rapidly to cryogenic temperatures in the presence of thermal hysteresis proteins. The integrity of the two-cell stage pig embryo was discussed earlier with respect to Fig. 5B.

The results of Figures 5 and 6 show that the addition of 40 mg/ml of thermal hysteresis protein dramatically improved the survival of the embryos and the oocytes, with 24.5% and 26% survival for the pig oocytes and pig embryos, respectively and 82.5% survival for the mouse embryos.

Because the mouse embryo provides such an unambiguous criteria for viability and because the survival in the presence of thermal hysteresis protein is so high, this animal model was particularly useful for the parametric studies. The results of the parametric studies on the effect of thermal hysteresis protein concentration are also listed in Table 2 above, and show a sudden transition between very high survival at concentrations higher than 20 mg/ml thermal hysteresis protein to very low, or no survival at concentrations lower than 10 mg/ml.

The results presented here clearly demonstrate that the thermal hysteresis proteins facilitate the survival of different animal models at cryogenic temperatures. The results also show that the mechanism of protection is associated with the ability of the thermal hysteresis proteins to maintain the integrity of the cell membrane during exposure to cryogenic temperatures. Thermal hysteresis proteins compounds that modify the process of freezing in solutions in a similar form to the thermal hysteresis proteins, sometimes have no effect on maintaining the structural integrity of the membrane.

### Figures 7A, 7B and 7C

Figures 7A, 7B and 7C show pig oocytes preserved for 4hr at 4°C without thermal hysteresis proteins (7A), and with 40m/ml thermal hysteresis proteins (7B and 7C). These figures show that the oolemma is damaged without the thermal hysteresis proteins (7A). It stays intact with thermal hysteresis proteins even in cells that do not survive, and it also facilitates the in vitro development of ooytes to the MII stage Figure (7C). The results in Table 2 suggest that the addition of thermal hysteresis proteins is useful in protecting cell membranes and in blocking ion channels. The observation that thermal hysteresis proteins 1-5 and 7-8 (Table 2, Fractions) separately do not protect the ion flow as well as the 1-5 and 7-8 together suggests that each one of the proteins is active in protecting different proteins and ion channels, i.e., is specific. Therefore it appears that all of the thermal hysteresis proteins are needed for complete protection, while individually, they offer partial protection.

### Figures 8 and 9

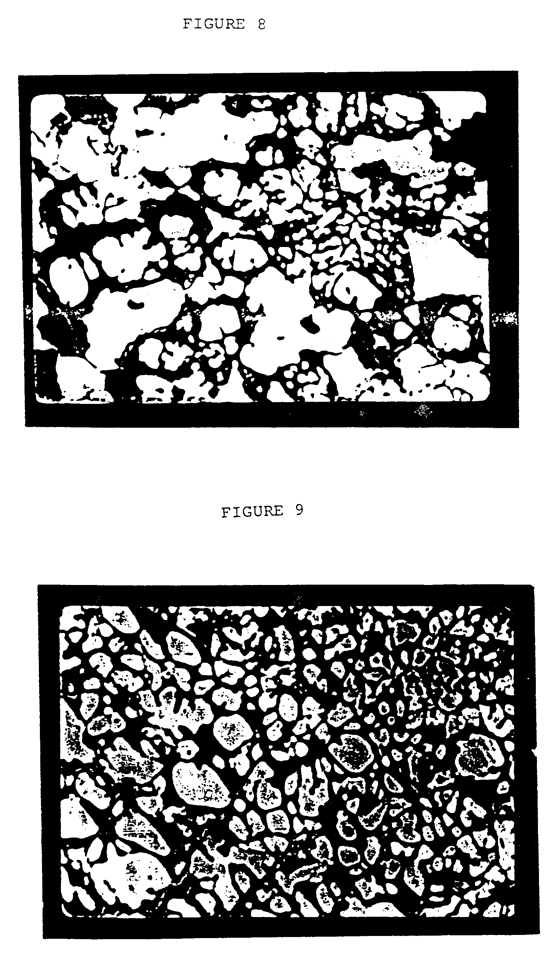

Figure 8 is a photogrpah of rat liver perfused with Krebs solution only and cooled to -35°C.

Figure 9 is a photograph of rat liver perfused with a identical Krebs solution for Fig. 8 with 20 mg/ml of AFGP fractions 1-8 as obtained from the Antarctic fish (see Table 1).

While not wanting to be bound by theory, the protective effect of the thermal hysteresis protein is probably associated with the particular chemical structure of the molecule. It is possible that the protection afforded to the cell membranes during exposure to cryogenic temperatures is a consequence of bonds formed between the hydrophilic parts of the membrane proteins and the thermal hysteresis proteins. There is evidence that the protection afforded by the thermal hysteresis proteins is concentration-dependent in a nonlinear fashion, which suggests that for complete protection all the bonds must be established and no survival is possible with partial interaction between the cell membrane and the thermal hysteresis protein.

The following examples are presented to further explain, describe and define the present invention. They are not to be construed to be limiting in any manner.

### GENERAL EXPERIMENTAL

The phosphate buffer solution (PBS) standard solution and may be supplemented as indicated herein, e.g. the higher molecular weight AFGPs 1 to 5 seems to be strongly related to the modification of the ice crystal structure, the biological function of the low molecular weight AFGPs remains unclear. They are less efficient in depressing the freezing point than the larger glycopeptides yet they seem to be present in the serum at much higher concentrations.

Thermal hysteresis protein used from Fractions 1-8 (Table 1) are in essentially the same ratio to each other as is found in the Antarctic fish. Fraction 6 is present in a trace amount in the fish, and its presence or absence in the following experiments (in the concentration of Fraction 1-8) is assumed to have a negligible effect on the experiment.

A preferred concentration of thermal hysteresis protein in aqueous solution in this invention ranges between about 1 and 50 mg/ml., especially Fraction 1-8, Table 1. For some applications, a range of 20-40 mg/ml is preferred.

As can be observed, ice crystal formation has caused major disruption of the blood vessels and the surrounding cell tissue and cell membrane.

As can be seen the cell membrane structure shows minimum disruption. The cell tissue appear to have remained discrete, the cell membrane appear essentially intact and the blood vessels are not significantly enlarged.

EXAMPLE 1

FREEZING OF LIVER TISSUE

(a) Adult female Sprague-Dawley rats, ages 45 to 50 days were anesthetized with ether throughout the surgical procedure. The abdomen was exposed via a midline incision to expose the liver. The portal vein was exposed and cannulated. Immediately, one thousand units of heparin were injected into the vein. This procedure was followed by the injection of a 5-ml solution of physiological saline containing 200 mg of thermal hysteresis protein from an Antarctic fish (Dissostichus Nawsoni) (see Table 1), in a physiological composition of Fractions 1-5 and 7,8 (25/75). Optionally, glycerol/saline is perfused through the liver. The thermal hysteresis proteins used are those shown in Table 1 above. The thermal hysteresis proteins for Dissostichus mawsoni and for the thermal hysteresis proteins of Table 1 have essentially the same molecular weights and ratios to one another. A combination of thermal hysteresis proteins No. 1-5 and No. 6-8 are used in a ratio of 1/3, w/w. The portal vein was immediately clamped to prevent back flow. Within a period of 2 minutes, several rectangular samples of the liver, 8 by 4 by 3 mm in size, were sectioned with single radial razor cuts approximately 3 mm from the periphery of the lobe and were placed lengthwise on two No. 1 coverslips. A total of four animal experiments were performed.

The first coverslip was immediately plunged into nitrogen slush maintained under vacuum at -213°C. No boiling was visible. The cooling rate during freezing was estimated at about 4000° C/min. At the same time, the other coverslip was transferred to the directional solidification stage described earlier. The samples were frozen from an initial temperature of 25° C to a final temperature of -35°C, with a cooling rate of 4°C/min. The time of freezing was approximately 15 min. After freezing, the frozen samples were immediately immersed in the liquid nitrogen slush and transferred to an AMRAY 1000 low temperature scanning electron microscope (LTSEM). The samples were fractured in the cryochamber of the microscope, exposing an area approximately 2 mm from the outer surface of the lobe, gold-coated and transferred in a frozen hydrated state to the refrigerated stage of the LTSEM.

Photographs obtained from the LTSEM are two-dimensional images of an irregularly fractured three-dimensional surface. The photographs are taken at magnifications varying from 200 to 5000 times.

Figures 2A, 2B and 2C show results from liver tissue perfused with thermal hysteresis proteins and frozen in nitrogen slush. These photographs demonstrate that the thermal hysteresis proteins modify the freezing pattern in mammalian tissue. Figures 2A and 2B illustrate the frozen tissue photographed at a magnification of 1000 times. Figures 2A and 2B were obtained after slight radiant etching of the frozen tissue, show the outline of ice crystals. Shown are individual cells and, in several of the cells, the nucleus is also visible. The ice crystals in the cells are different from those in the blood vessels. The ice crystals inside the cells are similar to typical ice crystals formed from plunging tissue in liquid nitrogen slush. These ice crystals are round in shape with dimensions in the micron range and are uniformly distributed throughout the cells. However, the ice crystals in the blood vessels perfused with thermal hysteresis proteins are markedly different. The ice crystal structure is spicular with dimensions in the submicron range. It is also very similar to that observed during freezing of aqueous solutions of thermal hysteresis proteins. See Figure 1C. Figures 2A, 2B, and 2C show spicular ice crystals in all the longitudinally and transversely fractured blood vessels and that the spicular ice crystals are oriented in the same direction independent of the relative orientation of the blood vessel.

These results demonstrate that water present in the tissue in the presence of thermal hysteresis proteins have ice crystals which do not propagate in the direction of the blood vessels but, rather, grow with a stable c-axis orientation, presumably in the direction of the temperature gradient. This is consistent with earlier reported research, which show that during freezing in a solution of thermal hysteresis proteins, the solutes entrapped between the spicules, stabilize and force the ice crystal to grow only in the direction of the c-axis. Therefore, the crystal growth is different from the freezing of solutions without thermal hysteresis proteins, where the ice crystal can grow along the different orientations of the ice crystal hexagonal prism facets, allowing a change of direction whenever the ice crystal encounters obstacles, such as the cell boundary. In the presence of the thermal hysteresis proteins, the growth in the direction of the c-axis is extremely stable and the orientation of the ice crystal cannot change when the ice crystal encounters a cell boundary. All the spicular ice crystals terminate at the blood vessel boundaries. The ice crystals in the blood vessels also do not cause the nucleation of the water in adjacent cells.

A higher magnification of a micrograph of the spicular ice crystals in a larger blood vessel is illustrated in Figure 2C. The significant difference between the spicular submicron size of the ice crystals in the blood vessel and the rounded micron size ice crystals in the adjacent cells in evident. The small dimensions of the spicular ice crystals suggest another potential application of the AFPs. Currently, extremely high cooling rates (e.g. 40,000 to 100,000°C/min, several orders of magnitude higher than the cooling rates in the present invention, are used for preparation of tissue samples with very small ice crystals for microscopy. Freezing tissues perfused with thermal hysteresis proteins in accordance with the present invention is used to produce small ice crystals in tissue with much lower cooling rates which can be easier to achieve experimentally.

Figures 3A, 3B, 3C, 3D and 3E demonstrate the effect of the thermal hysteresis proteins on the freezing pattern of mammalian tissue frozen with low cooling rates. The structure of liver tissue frozen with a cooling rate of 4°C/min in the presence of thermal hysteresis proteins is illustrated in Figures 3B, 3C, and 3D. These Figures are compared with Figure 3A which shows the structure of liver tissue frozen with a cooling rate of 4°C/min without AFPs and with Figure 3E which shows the structure of liver tissue frozen without AFPs with a cooling rate of approximately 4000°C/min.

(b) For comparison purposes Figure 3A shows the typical structure of liver tissue frozen at low cooling rates without thermal hysteresis proteins. The large continuous ice crystals along the sinusoids and the completely dehydrated hepatocytes surrounding the blood vessels are evident. Because of the dehydration of the hepatocytes in liver tissue frozen with low cooling rates, the tissue is not able to fracture along the cell membrane boundaries and, therefore, shows fractures through large ice crystals.

(c) The morphology of liver tissue frozen with low cooling rates in the presence of thermal hysteresis proteins is markedly different. Figures 3B and 3C show cross-sections through a large blood vessel and the adjacent tissue, at a magnification of 1000x and 2000x, respectively. The submicron size spicular ice structures, typically found in the freezing solutions with thermal hysteresis proteins, (Figure 1C) are evident in the blood vessel. All the ice crystals in the blood vessel have the same orientation and they terminate at the blood vessel boundary. The structure of the spicular ice crystals in Figures 3B and 3C are markedly different from the smooth single ice crystal structures observed in the blood vessels of tissue frozen with the same cooling rate but without AFPs, Figure 3A. Figures 2A, 2B, 2C, and 3A, 3B, 3C, 3D and 3E obtained for cooling rates of 4000°C/min and 4°C/min, respectively, demonstrate that the AFPs generate a similar, submicron size stable spicular ice crystal structure when freezing mammalian tissue over a large range of cooling rates.

(d) The fracture in Figures 3B, 3C, 3D and 3E is along the cell membrane, where the cells are removed in a staggered form, leaving behind stair-like arranged hepatocytes. The micrographs show contours of box-like shaped hepatocytes, which do not appear dehydrated and actually retain their normal shape. Comparing Figures 3B, 3C and 3D with Figures 3A and 3E, it is found that the figures possess much more resemblance to Figure 3E, showing identifiable box-like hepatocytes with typical dimensions, fractured at the cell membrane. Figure 3A which shows dehydrated hepatocytes and ice crystals in the expanded sinusoids, is significantly different. The surprising result is that while Figures 3B, 3C and 3D are photographs taken from samples perfused with thermal hysteresis proteins and frozen with a cooling rate of 4°C/min, which is similar to the cooling rate used for Figure 3A, Figure 3E is a photograph of liver tissue frozen, without thermal hysteresis proteins, in liquid nitrogen slush with a cooling rate of approximately 4000°C/min. As expected, freezing at these high cooling rates retains the normal structure of the hepatocytes, and shows the bile duct with bile along the cell membrane of Figure 3E. For freezing with low cooling rates (4°C/min) without thermal hysteresis proteins, it is impossible to fracture the dehydrated hepatocytes along the cell membrane, and these micrographs always show ice crystals. The observation is that the structure of liver tissue frozen with 4°C/min in the presence of thermal hysteresis proteins resembles that of tissue frozen with a cooling rate of 4000°C/ min. This result illustrates the significant effect of the thermal hysteresis proteins on the freezing pattern in mammalian tissue.

## EXAMPLE 2

## CRYOPRESERVATION OF WHOLE ORGAN

(a) Cryopreservation of a whole liver from a rat as described in Example 1 is adapted for a whole organ. The rat liver is surgically removed, and held in an aqueous solution at 24°C. The portal vein vessel is cannulated. The well known Langendorf perfusion system (with a first bottle containing Krebs solution) is used. See procedure, for example, D.E. Pegg et al. (1986), Cryobiology, Vol. 23, pp. 150-160.

A second bottle of solution contains a saline solution and appropriate quantities of glycerol, dimethyl sulfoxide, ethylene glycol, polyvinyl chloride glucose or mixtures of these substances which are known as protectants for cells together with antifreeze glycopeptides at a concentrations of 40 mg/ml.

A computer controlled mixing switch provides intermediate amounts of solution 1 (Krebs solution), solution 2 (glycerol/saline/AFGP solution) bursts of time lengths controlled by the computer, for example 0.01 sec. to 0.1 sec.

to 1 sec. time lengths. The two solutions mix in a specially provided mixing chamber.

These two bottles of physiological solution are connected to a mixing valve having known adjustable flow rates (e.g. about 5 ml/sec) and a computer to accurately vary the flow rate and mixing of each bottle's contents immediately prior to perfusion. The perfusion using the solutions of bottles 1 and 2 is well known in the art as described by G. N. Alink et al. (1976), Cryobiology, Vol. 13, pp. 295-304; (1977) Cryobiology, Vol. 14, pp. 409-417 and 399-408; and (1978) Cryobiology, Vol. 15, pp. 44-58, and K.E.F. Hobbs et al. (1969), Cryobiology, Vol. 6, pp. 239-245. The Krebs solution is perfused through the liver held at 24°C at a rate of 4 ml/min.

The glycerol/saline/AFGP concentration in the perfusate is slowly increased at a rate of 0.001-mole/0.1 sec. until a concentration of about 3 mol glycerol and 40m/ml AFGP is perfused. The tissue is then perfused with the 3 mol glycerol/saline for an additional 20 min. The perfused liver in solution (thermal hysteresis proteins 0.001 M in the organ) is next placed in a cooling stage (US Patent - 4,531,373) and the temperature of the whole perfused liver is then cooled at a rate of 1°C per minute until -150°C is achieved. The liver is then cooled using a liquid nitrogen slush to - 196°C and held at this temperature for 72 hrs. The frozen liver is then carefully thawed at a rate of between about 0.1 to 10°C per min. (preferably about 1°C per min.) using known techniques with warm fluids. Alternatively, carefully controlled microwave heating is used to thaw the perfused liver. When the thawed liver reaches about 0°C, a nutrient solution of Krebs is perfused through the large cannulated blood vessel. When warmed to 37°C, the thawed liver recovers not only tissue function but also organ function. The viability of the organ is measured by the production of bile following the freezing and careful thawing.

(b) When the rat liver in subpart (a) above is replaced with a rat kidney and the procedure is repeated, a thawed kidney having viable tissue function and recovered organ function is obtained.

(c) When the rat liver of subpart (a) is replaced with a rat heart, some additional procedures particular to heart tissue for perfusion, including immediate removal of blood from the heart chambers, are observed. After freezing of the thermal hysteresis protein perfused heart, careful thawing and perfusion with appropriate biological fluids, the reactivated heart having viable tissue function and viable organ function is obtained. The viability of the heart is measured by observing restored contractions of the heart muscle.

EXAMPLE 3

CRYOPROTECTION OF IMMATURE PIG OOCYTES AND PIG EMBRYOS

Immature pig oocytes were isolated from selected follicles of cyclic sows 20 minutes after slaughter at 20° C, according to the procedure by Mattioli, et al. (20). The two-cell stage pig embryos were collected from prepubertal gilts (average weight 90 kg.). Estrus induction was carried out by administration of 1250 I.U., Pregnant Mare Serum Gonadopin (PMSG), (SIGMA, St. Louis, Missouri), followed 56 hours later by administration of 750 I.U., Human Chorionic Gonadropin (HCG), SIGMA, St. Louis, Missouri). Two artificial inseminations were performed after 34 hours and 46 hours from the HCG injection. The two-cell embryos were collected from the animal by mid-ventral laparoscopy under general anesthesia 60 hours after the HCG injection.

In preparation for low temperature exposure, the embryos and the oocytes were first introduced in one ml of PBS containing 0.1 M sucrose and 20% FCS at 22° C. This was followed by a three-minute gradual mixing with one ml PBS containing 5% glycerol, 0.1 M sucrose and 35% propylene glycol, according to a procedure developed by Arav (21). The embryos and oocytes were transferred to slides containing 0.1 $\mu$l droplets of either AVS or AVS with thermal hysteresis proteins, one embryo or oocyte per droplet.

Prior to cooling, the pig oocytes and pig embryos were incubated on the slide for 6 minutes at 22° C.

Droplets containing oocytes and embryos were exposed in separate experiments, to the cooling/warming protocol described earlier. The cooling/warming process was monitored using a recording video camera attached to a Leitz Diaplan microscope with magnification of 120x and 340x. Fig. 4A illustrates the typical appearance of pig oocytes inside transparent droplets, during cooling with a rate of 1700° C/min to -130° C. In all experiments, the droplets remained transparent at magnification of 340x indicating the absence of visible ice crystals. During warming at 1,700° C/min, the transparent droplets retained an appearance identical to that in Figs. 4A and 6A. The microscopical evidence shows that the morphology of the embryos and oocytes did not change during cooling and warming.

After warming, in preparation for viability assays in cell culture, the pig oocytes and pig embryos were introduced for three minutes in 1 ml PBS, with 20% FCS and 1 M sucrose at room temperature (22° C), followed by transfer to and equilibration in PBS containing 20% FCS for 10 minutes at 22° C.

Prior to cell culture, all embryos and oocytes were washed three times in cell culture media. The pig oocytes were cultured in TCM-199 medium which was modified in that it contained 5 $\mu$g/ml of sheep luteinizing hormone (NIH S20), pig follicle stimulating hormone (LER 441-2) and 20 ng/ml of pig prolactin (LER 2073). The pig embryos were cultured in Brinster culture medium without glucose. After equilibration in cell culture medium, the oocytes and the embryos were incubated at 37° C under 5% $CO_2$ in air, the pig oocytes for 44 hours, and the pig embryos for 24 hours.

The pig oocytes were fixed after 44 hours incubation in acetic alcohol (1:3 v/v) and stained with lacmoid stain. The viability of immature pig oocytes was assessed using phase contrast microscopy (20), by their ability to develop from the germinal vesicular, (g.v.) stage to the first metaphase (MI) or second metaphase (MII) stage in vitro, and to present a normal morphology (cytoplasmatic compactness, integrated colemma, visible nuclear stage). The viability of the two-cell stage pig embryos was assessed by their ability to develop to the four-cell stage in culture, while maintaining integrated morphology (cell membrane and cytoplasm). The _in vitro_ culture was stopped at the four-cell stage because many times early stage pig embryos encounter the four-cell block when cultured in vitro (22), and therefore, further incubation would not be useful to an experimental goal to assess the viability of the embryos after exposure to cryogenic temperatures.

EXAMPLE 4

CRYOPROTECTION OF MOUSE EMBRYOS

The procedure of Example 3 was followed except for the following changes.

Mouse embryos at the two-cell stage, were obtained from four-week old $C_{57}Bl/GJ$ mice which were paired singly with CBA/CaJ males. The females were induced to superovulate by intraperitoneal injection of 5-7.5 I.U. PMSG (SIGMA, St. Louis, MO) followed 48 hours later by 5-7.5 I.U. HCG (SIGMA, St. Louis, MO). Forty hours after insemination, the oviducts were excised from the mice and the two-cell embryos flushed out and stored in phosphate buffeted saline, (PBS) medium.

In preparation for low temperature exposure the mouse embryos were introduced in 1 ml of PBS and FCS.

Prior to cooling, the mouse embryos were incubated on a slide for 12 min. at 4° C.

Fig. 6A illustrates the typical appearance of mouse embryos inside transparent droplets during cooling with a rate of 1,700° C/min to -130° C. In all experiments the droplets remained transparent at a magnification of 340x indicating the absence of visible ice crystals. During warming with 1700° C/min. the transparent droplets retained an appearance identical to that in Figs. 4A and 6A. The microscopical evidence shows that the morphology of the embryos and oocytes did not change during cooling and warming.

After warming, in preparation for viability assays in cell culture, as described in Example 3, the mouse embryos were exposed for three minutes at 4° C to 1 ml of PBS, with 20% FCS and 1 M sucrose, followed by transfer to and equilibration in PBS containing 20% FCS for 12 minutes at room temperature (22° C) (21). Prior to cell culture, all embryos were washed three times in cell culture media. The mouse embryos were cultured to $T_6$ Whittingham medium (21).

After equilibration in cell culture medium, the mouse embryos were incubated at 37° C under 5% $CO_2$ in air for 72 hours.

The viability of the mouse embryos after exposure to cryogenic temperatures was assessed by their ability to develop in vitro to the blastocyst stage while showing normal expanded morphology. The experimental results were discussed earlier.

EXAMPLE 5

CELL MEMBRANE ELECTRICAL POTENTIAL

During the experiments in which pig oocytes were vitrified in the presence of thermal hysteresis proteins, it was discovered that only 24% of the oocytes and 26% of the pig embryos survived the rapid cooling. However, it was surprisingly observed that close to 100% of the cell membranes remained intact. On the other hand, without the thermal hysteresis proteins (100%) of the cell membranes were destroyed. The effect of the thermal hysteresis proteins at temperatures higher than o° C for pig oocytes was examined because it is reported that they could not survive at temperatures lower than +10° C.

Immature pig oocytes were obtained from selected follicles of cylcic sows 20 minutes after slaughter, at 20°C, according to the procedure by Mattioli et al. The oocytes were then introduced in vials containing different concentrations of thermal hysteresis proteins (Fractions 1-8) in a saline supplemented with 0.4 w/v microm/ml BSA (Bovine Serum Albumin), 0.34 mM pyruvate, 5.5 mM glucose and 70 micromol/ml kanamycin). The thermal hysteresis proteins used in this work were obtained from Antarctic fish belonging to the family Notoheniidae (_Dissostichus Nawsoni_). A physiological composition of thermal hysteresis proteins was used in most of the experiments having one part by weight of thermal hysteresis proteins 1 to 5, and three parts by weight of thermal hysteresis proteins fractions 7 and 8 (available from A.L. DeVries _supra_.). Experiments were also performed with thermal hysteresis proteins fractions 1-5 and thermal hysteresis proteins 7 and 8, separately. To determine the protective effect of the thermal hysteresis proteins the oocytes were exposed for 4hr and 24hr to a constant temperature of 4°C, in a constant temperature chamber. After removing the oocytes from the 4°C environment the integrity of the oolema was determined by measuring the resting membrane potential of the oocytes at room temperature, 22°C, according to a procedure by Mattioli et al. Intracellular voltage measurements were made using single microelectrodes made from borosilicate glass tubes (Hilgenberg, FDR). The electrodes were pulled on a horizontal puller and filled with 2M KC1. The resistance of the electrodes was 10-20 Megaohms (Mo). To record the membrane potential the tip of the microelectrode was maneuvered to the surface of the cell using a micromanipulator controlled through 400x magnification with a Leitz Fluovert microscope equipped with Nomarski optics. When the tip just dimpled the surface of the cell, the final penetration was achieved by briefly causing an electrical oscillation induced by turning the capacity compensation of the amplifier. The electrical potential values, which remained constant for at least 1-2 sec, were recorded. The resting membrane potential is a very sensitive criteria for membrane integrity. The results were obtained and are shown in Table 2 above.

In addition, experiments were performed to determine the viability of certain oocytes following exposure to the hypothermic conditions. Several of the oocytes exposed for 4 hr to 4°C, with 40mg/ml AFGP 1-8 in the basic PBS solution and without, were incubated in TCM-199 medium, which was modified in that it contained 5 microg/ml sheep luteinizing hormone (NIH S20), pig follicle stimulating hormone (LER 441-2) and 20 nanog/ml of pig prolactin (LER 2073) at 37°C under 5% carbondioxide for 44 hr. After incubation the oocytes were fixed in acetic acid/ethyl alcohol (1:2 v/v) and stained by lacmoid stain. The viability of the immature pig oocytes was assessed using phase contrast microscopy by their ability to develop from the initial germinal vesicular (g.v.) stage to the first or second metaphase, MI or MII in vitro and to present a normal morphology (cytopolasmatic compactness, integrated oolema, visible nuclear stage). The microscopic observation also allows a qualitative evaluation of the structural integrity of the membrane.

The mean of all means was -31 mv, and the mean standard distribution, 4.5 mv. These values are within the normal range of membrane potential for pig oocytes. It should be emphasized that measuring potential is a very sensitive and recognized measure of membrane integrity. It was apparent from the results shown in Table 2 that the combined thermal hysteresis protein fractions 1-8 protect the olema against damage induced by exposing the oocytes to hypothermic conditions. Since there are no ice crystals present at 4°C the protection must occur through an interaction between the Antarctic fish glycoproteins and the oolema. Therefore, this part of the experiment demonstrates that the thermal hysteresis proteins directly protect membranes, which is a property of the thermal hysteresis proteins that has never been reported. The level of protection is not a linear function of the thermal hysteresis protein concentration; it reaches saturation at about 1 mg/ml in the perfusion and drops to low values at 0.1 mg/ml. This is a typical property of protein-protein interactions, which may indicate that the glycoproteins may offer protection by binding to the available sites on the oolema and can provide their protection only if all the sites are occupied. These sites could be the membrane proteins. Table 2 also shows that the whole physiological combination of thermal hysteresis proteins 1 to 8 is needed for protection and that thermal hysteresis proteins 5 and 7, 8 separately do not protect the membrane. This result is extremely surprising because studies on the effects of thermal hysteresis proteins on depressing the phase transition temperature and ice crystal formation show that thermal hysteresis protein fractions 1-5 depress the phase transition temperature almost as effectively as the whole combination of thermal hysteresis proteins fractions 1 to 8. On the other hand, it is apparent that thermal hysteresis proteins fractions 1 to 5 separately do not protect the cell membrane, and neither do thermal hysteresis proteins fractions 7, 8 separately. A possible explanation for this phenomena is that all the different proteins with different lengths are needed to bind to all possible sites on the membrane and to block all the possible leaks sites and ions channels.

The microscope evaluations of the oocytes exposed to 4°C for 4 hr and incubated for 44 hr to verify the results obtained through measurements of membrane resting potential. In the absence of thermal hysteresis proteins, only 2 of 20 oocytes retained an integrated oolemma (10%), and none of the oocytes matured in vitro, (0%). Figure 7A illustrates the appearance of an oocyte preserved at 4°C in PBS without thermal hysteresis protein. The oolema is apparently not integrated and the cytoplasm is degenerated. In contrast, in the presence of 40 mg/ml thermal hysteresis protein, 11 of 18 oocytes retained an integrated oolemma, 61% (Figure 7B). This result further demonstrates that the thermal hysteresis proteins protect the oolema of cells exposed to damaging hypothermic conditions and is consistent with the measurements of the resting electrical potential. Close to 25% of the oocytes survived and matured to the MII stage as illustrated by (Figure 7C).

EXAMPLE 6

CRYOPRESERVATION EFFECT OF THE THERMAL HYSTERESIS PROTEINS ON RAT LIVER

The procedure is essentially identical to that described in Example 1.

Experiments were performed with adult female Sprague-Dawley rats, ages 45 to 50 days. The rats were anesthetized with ether throughout the surgical procedure. The abdomen was exposed via a midline incision to expose the liver. The bile duct was exposed and cannulated. Bile was collected and used as a criteria for viability. The portal vein was exposed and cannulated. Immediately one thousand units of heparin were injected into the vein. The liver was released and flushed with a basic Krebs solution through the vein. In some of the models this was followed by an injection through the vein of 3 ml solution of Krebbs containing 20 mg/ml thermal hysteresis proteins Fractions 1-8 (in a physiological composition found in the fish) (from the Antarctic fish belonging to the family Nototheniid, Dissostichus Mawsoni). The liver was then introduced into a refrigerator at 4° C for 6 hours. After that time, the liver was removed, perfused with a Krebbs solution at body temperature and kept on a plate maintained at body temperature 38°C. The production of bile was measured as a criteria for viability. This test is a well accepted criteria considered to provide the best overall indication of viability. The results show that with thermal hysteresis proteins, the bile rate of formation was about 85% of the initial level after 6 hours at 4° C. In the absence of the thermal hysteresis protein, bile rate dropped to about 20% of the normal level. Three animal experiments were performed for both the control and the solution with thermal hysteresis protein.

## EXAMPLE 6A

## EFFECT OF THERMAL HYSTERESIS PROTEIN ON LONG-TERM VIABILITY OF INTACT RAT LIVER TISSUE

### Surgical Procedure

Livers from Spague-Dawley rats (35) of ages 50-55 days are surgically removed. The peritoneal cavity is entered under nembutal anesthesia. The bile duct is cannulated with a PE-30 polyethylene catheter, and the bile is collected for 10 min while the surgery proceeds. After partial mobilization of the liver from adjacent tissue, a 16-gauge TEFLON® intravenous catheter is introduced into the portal vein, and 3.0 mL of perfusion buffer containing 1000 units of heparin is rapidly infused using a 3 mL syringe. The inferior vena cava is transected distally, and the portal vein catheter infused with Krebs solution pre-equilibrated to a 95:5 mixture of $O_2$ and $CO_2$ at 0°C from the remainder of the surgical procedure. The inferior vena cava is ligated above the renal veins and freed from adjacent retroperitoneal tissues, and a PE-205 polyethylene catheter is secured in the superior vena cava through an incision in the right atrium. Samples of the effluent are collected at this time. The entire liver is then carefully cut free from the surrounding tissue and washed with warm saline.

### Storage and Isolated Organ Perfusion

For the test livers, the perfusion line of TEFLON® is removed and a 3 mL solution of Krebs solution containing 20 mg/mL of AFGP fraction 1-8 from Antarctic notothenoiidae fish (D. Mawsoni) is injected into the catheter. The whole liver is then immediately placed into a container containing cold Krebs solution and is returned to the constant temperature apparatus. The apparatus and liver are kept at a constant 4°C. The liver is stored for periods of 6, 12 and 24 hr.

After the storage process, the liver is removed and inject with a 20 mL of Krebs solution at ambient temperature to remove the thermal hysteresis protein solution. The liver is immediatel inserted in the single pass Largends rf type perfusion circuit described by containing Krebs solution 37°C pre-equilibrated with a mixture of 95% oxygen and 5% carbon dioxide. The flow rate is then increased from 5 to 25 mL/min with careful attention to the position of the liver and catheter. The liver is perfused for 50 min. The effluent from the liver is collected continuously for the intervals 0-5, 5-10 and 10-25 min. In addition, bile is collected in 15 min intervals.

### Control

For a stored liver control study, the liver was injected with a 3 mL of Krebs solution. The procedure then followed the test study conditions described above without AFGP addition. For the warm control liver, the liver was immediately inserted in the single pass perfusion circuit, and the necessary effluent and bile samples was taken.

The collected bile from each liver was measured and tabulated. The collected effluent was tested for activity of lactic dehydrogenase (LDH). Enzyme assays for LDH is performed using standard colorimetric techniques (Sigma Diagnostics KIT 500. A UV-visible spectrophotometer is utilized.

Experiments were performed with 35 rats. Each experimental point represents between 3 and 5 animal experiments.

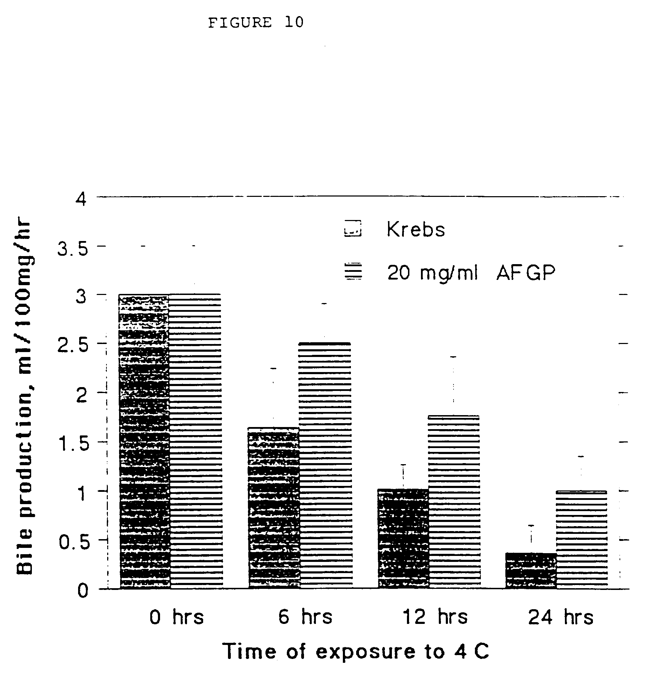

Bile flow commenced within 3-5 mins. after the liver was connected to the single pass perfusion system. The bile flow was well maintained during the 50 min perfusion. Since rat livers do not produce bile salt, the production of bile from any excised liver could not be sustained for much longer than about 50 minutes. The production of bile reaches a plateau during the second collection and maintains this level for the duration of the perfusion. Figure 10 is a plot of the bile production from the second collection versus the storage period 6, 12 and 24 hrs. Figure 10 shows the gradual decrease of bile production as the storage period increases. The solid line column represents the bile production from liver stored with AFGP in Krebs solution. The dashed line column represents livers stored with Krebs solution only.

There is a significant increase in bile production from livers stores with the thermal hysteresis protein at all the storage times. Although the bile flow decreased significantly after 24 hr. of storage, the bile production with AFGP showed an improvement over liver stored with Krebs solution only.

In the 24 hr storage experiments with thermal hysteresis protein, the typical LDH activities during the perfusion process is shown by the solid line column in Figure 10. The dotted line column represent the results from the control liver perfusion, and the liver stored in Krebs solution are represented by the dashed line column. For the thermal hysteresis protein perfused stored liver, the release of the enzymes is at a maximum during the first 5 min of the perfusion, and then decreases at later times to near control levels. The Krebs solution stored liver also reaches a maximum during the first 5 min. of perfusion. However, the decrease in activity level at later times remains significantly higher than control levels.

In utilizing the enzyme colorimetric tests, these results from LDH activity show the membrane protective capability of the thermal hysteresis protein. Since leakage of higher levels of LDH activity is reported to be associated with membrane damage, this test provides an indication of the integrity of the hepato cellular membrane. The results of the LDH tests show a significant decrease in activity for livers stored in thermal hysteresis protein, which indicates lessor damage to the cellular membrane. From these results, AFGP clearly provides cellular membrane protection during the storage process. As indicated by the increase in bile production, the AFGP protection of membrane leads to better preservation capability compared to Krebs solution alone.

### EXAMPLE 6B

### PRESERVATION OF RABBIT HEART

A preliminary experiment and a control experiment were performed in parallel using adult rabbit heart.

Two white laboratory rabbits (2-3 kg each) were anesthetized. Each heart was surgically removed. The control heart was perfused with Krebs solution for 30 sec. at 5°C and injected with 5 ml of standard Krebs solution (5°C). The other heart was perfused with Krebs solution for 30 sec. at 5°C. The aorta chamber was injected with a standard Krebs solution (at 5°C) containing 20 mg/ml of thermal hysteresis proteins - Fractions 1-5 and 7-8 (Table 1) (25/75, w,w) purified by standard electrophoreses. Each heart was immediately placed in a small test tube containing Krebs solution at 5°C and placed into an ice/water bath at about 0°.

The control heart (without thermal hysteresis protein) was held at 0°C for 4 hr, then connected to a Langendorf perfusion system, and perfused with Krebs solution at 37°C (optionally containing some glucose) for one hr. During this time, the aorta beat weakly (or fluttered). At the end of one hour at 37° (physiological temperature), the aortic pressure was about 27 mm of water, aortic flow was negligible, and the flow rate through the heart was about 2 cc/min. This heart was nonvigorous, beating at about 30 beats per minute. Visually the portions of the heart tissue appeared to be dead or dying.

The experimental heart (with thermal hysteresis protein) was also held at 0°C for 4 hr, then connected to a Langendorf perfusion apparatus and perfused with Krebs solution at 37°C (optionally containing glucose). At the end of one hour at 37°C, the aortic pressure was over 100 mm water, aortic flow was 12 cc/min, and a cardiovascular flow rate of about 47 cc per min. was measured. This heart was vigorous, beating about 160 beats per minute. Visually, the heart looked robust, these measurements were close to the values for a normal heart.

### EXAMPLE 7

Mouse embryos at the two cell stage were introduced in a T6 Whittingham medium with 40 mg/ml antifreeze glycopeptides and without an atmosphere of 5% $CO_2$ and a temperature of 37°C for 72h. Accidentally the concentration of $CO_2$ increased to about 8% and the temperature fluctuated while being most of the time at temperatures higher than 40°C. After incubation at these non optimal environmental conditions close to 80% of the mouse embryos developed to the blastocyst stage in the presence of the antifreeze protein while less than 50% developed to the blastocyst stage without the antifreeze protein. This result demonstrates another useful property of the thermal hysteresis proteins at hyperthermic temperatures that is not compatible with the cells.

### Claims

1. A method for the protection and preservation of the viability of biological material selected from mammalian cells, tissue or organs undergoing exposure to a non-physiological temperature; said method comprising contacting said biological material with a biologically compatible solution of at least one thermal hysteresis protein before exposure to said non-physiological temperature, wherein said thermal hysteresis protein is obtainable from Antarctic notothenoids, Channichthyidae, Bathydrconidae, Northern ocean gadoids, Pleuroectinae such as righteye flounders, cottids, and Zoarcoidei (eel pouts).

2. A method according to claim 1 wherein the biologically compatible solution is an aqueous solution.

3. A method according to claim 1 or claim 2 wherein the non-physiological temperature is a hypothermal temperature of 4°C or below.

4. A method according to claim 1 or claim 2 wherein the non-physiological temperature is a hyperthermal temperature higher than 40°C.

5. A method according to any one of the preceding claims wherein the thermal hysteresis protein is a glycopeptides obtainable from the Antarctic nototheniidae fish, Northern ocean gaddoids, the winter flounder, the Alaskan plaice, the Atlantic sculpin, the Grubby sculpin or the Antarctic eel pout.

6. A method according to claim 5 wherein the thermal hysteresis protein is a glycopeptide obtainable from the Antarctic nototheniidae fish, or the Antarctic eel pout of the Arctic winter flounder.

7. A method according to claim 6 wherein the thermal hysteresis protein is a glycopeptide obtainable from the species Dissostichus Mawsoni, Pagoothenia Borchgnevinki, or Rhigophile dearborni.

8. A method according to any one of the preceding claims wherein the thermal hysteresis protein is identical to at least one of (a) fractions 1 - 5, (b) 6, and (c) 7 and 8 of the a glycopeptide obtainable from the Antarctic Notheniidae fish, wherein fraction 1 has a molecular weight of 33,700 Daltons, fraction 2 has a molecular weight of 28,800 Daltons, fraction 3 has a molecular weight of 21,500 Daltons, fraction 4 has a molecular weight of 17,000 Daltons, fraction 5 has a molecular weight of 10,500 Daltons, fraction 6 has a molecular weight of 7,900 Daltons, fraction 7 has a molecular weight 3,500 Daltons and fraction 8 has a molecular weight of 2,600 Daltons.

9. A method according to claim 8 wherein, the antifreeze glycopeptide comprises fractions 1 - 5 about 25% by weight and fractions 7 and 8 about 75% by weight.

10. A method according to any one of the preceding claims wherein the thermal hysteresis protein comprises either

(a) a peptide having multiple regions of alanine-alanine-threonine- or alanine-alanine-alanine-, or
(b) a glycopolypeptide having multiple regions of alanine-alanine-threonine, wherein $\beta$-D-galactopyranosyl-(1-3)-2-acetamide-2-deoxy-$\alpha$-D-galactopyranose is covalently attached to substantially all of the threonine residues.

11. A method according to claim 9 wherein the molecular weight of the polypeptide or glycopeptide is between about 2,000 and 50,000 daltons.

12. A method according to claim 11 wherein the molecular weight of the glycopeptide is between about 2,200 and 40,000 daltons.

13. A method according to any one of the preceding claims wherein the biologically compatible solution comprises between 0.1 and 100mg/ml of thermal hysteresis protein.

14. A method according to claim 13 wherein the biologically compatible solution comprises between 1 and 60 mg/ml of thermal hysteresis protein.

15. A method according to any one of the preceding claims wherein the biologically compatible solution comprises an additional preserving, protecting or vitrifying compound selected from glycerol, dimethyl sulfoxide, ethylene glycol, polyvinyl pyrrolidine, glucose, sucrose, propanediol, propylene glycol, carboxymethyl cellulose, or mixtures of these agents which are known to protect cells and biological materials against freezing damage or to promote vitrification.

16. A method according to any one of the preceding claims wherein the thermal hysteresis protein has alternating hydrophobic and hydrophilic regions which repeat between each 16-17 or 19-20 Angstroms, or each 16.5 or 19.5 Angstroms.

17. A method according to any one of the preceding claims for wherein the non-physiological temperature is a hypothermal temperature of 0°C or lower.

18. A method according to claim 17 which further includes the steps of removing the thermal hysteresis protein and returning the biological material to a physiological temperature and composition, and obtaining the biological material having renewed physiological viability and functionality.

19. A method according to any one of the preceding claims wherein the biological material is subject to a temperature of from 0°C (273K) to -269°C (4K).

20. A method according to claim 18 wherein the biological material is also contacted with a cryoprotective compound prior to exposure to cryogenic temperatures under conditions resulting either in freezing or in vitrification.

21. A method according to any one of the preceding claims wherein the biological material is mammalian tissue or organ and the biologically compatible solution is perfused through the vasculature of the tissue or organ.

22. The method according to claim 21 herein the concentration of the thermal hysteresis protein is present in between about 1 and 50mg/ml of solution within blood vessels of the material.

23. A method according to claim 4 which further includes the steps of :

> warming the system by warming means including warm fluids or microwave heating to physiological temperature conditions and
> removing thermal hysteresis protein and any other cryo protective compounds and replacing them by physiological compatible solutions; and
> obtaining the biological material having physiological viability and functionality.

24. The use of a thermal hysteresis protein in a method according to claim 1 for the protection and preservation of the viability of animal cell membranes, animal cells, tissues or organs.

**Patentansprüche**

1. Verfahren zum Schützen und Konservieren der Lebensfähigkeit von biologischem Material, ausgewählt aus Säugetierzellen, -gewebe oder -organen, das einer nicht-physiologischen Temperatur ausgesetzt wird, wobei das Verfahren das In-Kontakt-Bringen des biologischen Materials mit einer biologisch verträglichen Lösung zumindest eines thermischen Hystereseproteins umfaßt, bevor es der nicht-physiologischen Temperatur ausgesetzt wird, worin das thermische Hystereseprotein von Antarktis-Notothenoiden, Channichthyidae (Eisfischen), Bathydraconidae (Drachenfischen), Nordsee-Gadoiden (-Schellfischen), Pleuronectidae (Plattfischen), wie z.B. Rechtsaugen-Flundern, Cottidae (Groppen) und Zoarcoidei (Aalmuttern, Hammelfleischfischen), erhältlich ist.

2. Verfahren nach Anspruch 1, worin die biologisch verträgliche Lösung eine wäßrige Lösung ist.

3. Verfahren nach Anspruch 1 oder 2, worin die nicht-physiologische Temperatur eine hypothermale Temperatur von 4°C oder darunter ist.

4. Verfahren nach Anspruch 1 oder 2, worin die nicht-physiologische Temperatur eine hyperthermale Temperatur von über 40°C ist.

5. Verfahren nach einem der vorangegangenen Ansprüche, worin das thermische Hystereseprotein ein Glykopeptid ist, das von Antarktis-Nototheniidae, Nordsee-Gadoiden (-Schellfischen), der Winterflunder, der Alaska-Scholle, dem Atlantik-Seeskorpion, dem Grubby-Seeskorpion oder der Antarktis-Aalmutter erhältlich ist.

6. Verfahren nach Anspruch 5, worin das thermische Hystereseprotein ein Glykopeptid ist, das von Antarktis-Nototheniidae oder der Antarktis-Aalmutter oder der Arktis-Winterflunder erhältlich ist.

7. Verfahren nach Anspruch 6, worin das thermische Hystereseprotein ein Glykopeptid ist, das von der Spezies Dissostichus Mawsoni, Pagoothenia Borchgnevinki oder Rhigophile dearborni erhältlich ist.

8. Verfahren nach einem der vorangegangenen Ansprüche, worin das thermische Hystereseprotein mit zumindest einem von (a) den Fraktionen 1 bis 5, (b) Fraktion 6 und (c) den Fraktionen 7 und 8 des a-Glykopeptids identisch ist, das von Antarktis-Nototheniidae erhältlich ist, wobei

Fraktion 1 ein Molekulargewicht von 33.700 Dalton aufweist,
Fraktion 2 ein Molekulargewicht von 28.800 Dalton aufweist,
Fraktion 3 ein Molekulargewicht von 21.500 Dalton aufweist,
Fraktion 4 ein Molekulargewicht von 17.000 Dalton aufweist,
Fraktion 5 ein Molekulargewicht von 10.500 Dalton aufweist,
Fraktion 6 ein Molekulargewicht von 7.900 Dalton aufweist,
Fraktion 7 ein Molekulargewicht von 3.500 Dalton aufweist und
Fraktion 8 ein Molekulargewicht von 2.600 Dalton aufweist.

9. Verfahren nach Anspruch 8, worin das Frostschutz-Glykopeptid zu etwa 25 Gew.-% die Fraktionen 1 bis 5 und zu etwa 75 Gew.-% die Fraktionen 7 und 8 umfaßt.

10. Verfahren nach einem der vorangegangenen Ansprüche, worin das thermische Hystereseprotein entweder

(a) ein Peptid mit mehreren Regionen von Alanin-Alanin-Threonin- oder Alanin-Alanin-Alanin- oder
(b) ein Glykopolypeptid umfaßt, das mehrere Regionen von Alanin-Alanin-Threonin- aufweist, worin β-D-Galactopyranosyl-(1-3)-2-acetamid-2-desoxy-α-D-galactopyranose kovalent an im wesentlichen alle Threoninreste gebunden ist.

11. Verfahren nach Anspruch 9, worin das Molekulargewicht des Polypeptids oder Glykopeptids zwischen etwa 2.000 und 50.000 Dalton liegt.

12. Verfahren nach Anspruch 11, worin das Molekulargewicht des Glykopeptids zwischen etwa 2.200 und 40.000 Dalton liegt.

13. Verfahren nach einem der vorangegangenen Ansprüche, worin die biologisch verträgliche Lösung zwischen 0,1 und 100 mg/ml thermisches Hystereseprotein umfaßt.

14. Verfahren nach Anspruch 13, worin die biologisch verträgliche Lösung zwischen 1 und 60 mg/ml thermisches Hystereseprotein umfaßt.

15. Verfahren nach einem der vorangegangenen Ansprüche, worin die biologisch verträgliche Lösung eine zusätzliche Konservierungs-, Schutz- oder Vitrifizierungsverbindung umfaßt, die aus Glycerin, Dimethylsulfoxid, Ethylenglykol, Polyvinylpyrrolidon, Glucose, Saccharose, Propandiol, Propylenglykol, Carboxymethylzellulose oder Gemischen dieser Mittel ausgewählt ist, von denen bekannt ist, daß sie Zellen und biologische Materialien gegen Frostschäden schützen oder die Vitrifizierung fördern.

16. Verfahren nach einem der vorangegangenen Ansprüche, worin das thermische Hystereseprotein alternierend hydrophobe und hydrophile Bereiche aufweist, die sich jeweils zwischen 16-17 oder 19-20 Ångström oder jeweils 16,5 oder 19,5 Ångström wiederholen.

17. Verfahren nach einem der vorangegangenen Ansprüche, worin die nicht-physiologische Temperatur eine hypothermale Temperatur von 0°C oder darunter ist.

18. Verfahren nach Anspruch 17, das weiters die Schritte des Entfernens des thermischen Hystereseproteins und des Zurückbringens des biologischen Materials auf physiologische Temperatur und Zusammensetzung sowie des Erhaltens des biologischen Materials mit erneuerter physiologischer Lebensfähigkeit und Funktionalität umfaßt.

19. Verfahren nach einem der vorangegangenen Ansprüche, worin das biologische Material einer Temperatur von 0°C (273K) bis -269°C (4K) ausgesetzt wird.

20. Verfahren nach Anspruch 18, worin das biologische Material auch mit einer Kälteschutzverbindung in Kontakt gebracht wird, bevor es unter Bedingungen, die entweder zum Frieren oder zur Vitrifizierung führen, Tieftemperaturen ausgesetzt wird.

21. Verfahren nach einem der vorangegangenen Ansprüche, worin das biologische Material Säugetiergewebe oder -organ ist und die biologisch verträgliche Lösung durch das Gefäßsystem des Gewebes oder Organs strömen gelassen wird.

22. Verfahren nach Anspruch 21, worin die Konzentration, in der das thermische Hystereseproteins in den Blutgefäßen des Materials vorhanden ist, zwischen 1 und 50 mg/ml Lösung beträgt.

23. Verfahren nach Anspruch 4, das weiters folgende Schritte umfaßt:

das Erwärmen des Systems durch Erwärmungsmittel, einschließlich von warmen Fluids oder Mikrowellenheizung, auf physiologische Temperaturbedingungen, und
das Entfernen des thermischem Hystereseproteins und jeglicher anderer Kälteschutzverbindungen und Ersetzen derselben durch physiologisch verträgliche Lösungen; sowie
das Erhalten des biologischen Materials mit physiologischer Lebensfähigkeit und Funktionalität.

24. Verwendung eines thermischen Hystereseproteins in einem Verfahren nach Anspruch 1 zum Schutz und zur Konservierung der Lebensfähigkeit von Tierzellmembranen, Tierzellen, Geweben oder Organen.

**Revendications**

1. Méthode pour la protection et la conservation de la viabilité d'un matériel biologique sélectionné parmi des cellules, du tissu ou des organes mammaliens subissant une exposition à une température non physiologique, ladite méthode comprenant la mise en contact dudit matériel biologique avec une solution biologiquement compatible d'au moins une protéine à hystérésis thermique avant exposition à ladite température non physiologique où ladite protéine à hystérésis thermique peut être obtenue des notothénidés de l'Antarctique, des Channichthyidés, des Bathycrinidés, des gadides des océans du Nord, des pleuronectidés comme le carrelet à yeux à droite, les cottidés, et les zoarcoidés (lottes).

2. Méthode selon la revendication 1 où la solution biologiquement compatible est une solution aqueuse.

3. Méthode selon la revendication 1 ou la revendication 2 où la température non physiologique est une température hypothermique de 4°C ou moins.

4. Méthode selon la revendication 1 ou la revendication 2 où la température non physiologique est une température hyperthermique plus haute que 40°C.

5. Méthode selon l'une quelconque des revendications précédentes où la protéine à hystérésis thermique est un glycopeptide obtenu du poisson notothéniidé de l'Antarctique, des gadidés des océans du Nord, du carrelet d'hiver, de la plie de l'Alaska, du callionyme de l'Atlantique, du callionyme crasseux ou de la lotte de l'Antarctique.

6. Méthode selon la revendication 5 où la protéine à hystérésis thermique est un glycopeptide pouvant être obtenu du poisson notothéniidé de l'Antarctique, ou de la lotte de l'Antarctique ou du carrelet d'hiver de l'Arctique.

7. Méthode selon la revendication 6 où la protéine à hystérésis thermique et un glycopeptide pouvant être obtenu de l'espèce <u>Dissostichus Mawsoni</u>, <u>Pagoothenia Borchgnevinki</u>, ou <u>Rhigophile dearborni</u>.

8. Méthode selon l'une quelconque des revendications précédentes où la protéine à hystérésis thermique est identique à au moins l'une de (a) fractions 1-5, (b) 6, et (c) 7 et 8 du glycopeptide pouvant être obtenu du poisson Notothéniidé de l'Antarctique, où la fraction 1 a un poids moléculaire de 33 700 daltons, la fraction 2 a un poids moléculaire de 28 800 daltons, la fraction 3 a un poids moléculaire de 21 500 daltons, la fraction 4 a un pois moléculaire de 17 000 daltons, la fraction 5 a un poids moléculaire de 10 500 daltons, la fraction 6 a un poids moléculaire de 7 900 daltons, la fraction 7 a un poids moléculaire de 3 500 daltons et la fraction 8 a un poids moléculaire de 2 600 daltons.

9. Méthode selon la revendication 8 où le glycopeptide antigel comprend les fractions 1-5 à raison d'environ 25% en poids et les fractions 7 et 8 à raison d'environ 75% en poids.

10. Méthode selon l'une quelconque des revendications précédentes où la protéine à hystérésis thermique comprend soit

(a) un peptide ayant des régions multiples d'alanine-alanine-thréonine- ou alanine-alanine-alanine-, ou

(b) un glycopolypeptide ayant des réglos multiples d'alanine-alanine-thréonine- ou le β-D-galactopyranosyl-(1-3)-2-acétamide-2-désoxy-α-D-galactopyranose est attaché de manière covalente à sensiblement tous les résidus de thréonine.

11. Méthode selon la revendication 9 où le poids moléculaire du polypeptide ou du glycopeptide est compris entre environ 2000 et 50 000 daltons.

12. Méthode selon la revendication 11 où le poids moléculaire du glycopeptide est compris entre environ 2 200 et 40 000 daltons.

13. Méthode selon l'une quelconque des revendications précédentes où la solution biologiquement compatible comprend entre 0,1 et 100 mg/ml de la protéine à hystérésis thermique.

14. Méthode selon la revendication 13 où la solution biologiquement compatible comprend entre 1 et 60 mg/ml de la protéine à hystérésis thermique.

15. Méthode selon l'une quelconque des revendications précédentes où la solution biologiquement compatible comprend un composé additionnel de conservation, protection ou vitrification sélectionné parmi le glycérol, le diméthyl sulfoxyde, l'éthylène glycol, la polyvinyl pyrrolidone, le glucose, le saccharose, le propanediol, le propylène glycol, la carboxyméthyl cellulose, ou des mélanges de ces agents qui sont connus pour protéger les cellules et les matériaux biologiques contre les dégâts dûs au gel ou pour favoriser la vitrification.

16. Méthode selon l'une quelconque des revendications précédentes où la protéine à hystérésis thermique a des régions hydrophobes et hydrophiles alternées qui se répètent entre chaque 16-17 ou 19-20 angstroms, ou chaque 16,5 ou 19,5 angstroms.

17. Méthode selon l'une quelconque des revendications précédentes où la température non physiologique est une température hypothermique de 0°C ou moins.

18. Méthode selon la revendication 17 qui comprend de plus les étapes d'éliminer la protéine à hystérésis thermique et de remettre le matériau biologique à une température et une composition physiologique et d'obtenir le matériel biologique ayant une viabilité et une fonctionnalité physiologiques renouvelées.

19. Méthode selon l'une quelconque des revendications précédentes où le matériel biologique est soumis à une température de 0°C (273K) à -269°C (4K).

20. Méthode selon la revendication 18 où le matériel biologique est également mis en contact avec un composé cryoprotecteur avant exposition à des températures cryogéniques dans des conditions ayant pour résultat une congélation ou une vitrification.

21. Méthode selon l'une quelconque des revendications précédentes où le matériel biologique est du tissu ou organe mammalien et la solution biologiquement compatible est perfusée à travers la vascularisation du tissu ou organe.

22. Méthode selon la revendication 21 où la concentration de la protéine à hystéréris thermique est présente entre environ 1 et 50 mg/ml de la solution dans les vaisseaux sanguins du matériel.

23. Méthode selon la revendication 4 qui comprend de plus les étapes de :

chauffer le système par un moyen chauffant comprenant des fluides chauds ou un chauffage à micro-ondes aux conditions de température physiologique et

éliminer la protéine à hystérésis thermique et tout autre composé cryoprotecteur et les remplacer par des solutions physiologiques compatibles; et

obtenir le matériel biologique ayant une viabilité et une fonctionnalité physiologique.

**24.** Utilisation d'une protéine à hystérésis thermique dans une méthode selon la revendication 1 pour la protection et la conservation de la viabilité des membranes de cellules animales, des cellules animales, des tissus ou organes.

FIGURE I A

FIGURE 1B

FIGURE 1C

FIGURE 2 A

FIGURE 2 B

FIGURE 2 C

FIGURE 3A

FIGURE 3 B

FIGURE 3C

FIGURE 3D

FIGURE 3 E

FIGURE 4A

FIGURE 4B

FIGURE   4C

FIGURE   4D

FIGURE
5A

FIGURE 5B

FIGURE 6A

FIGURE
6B

FIGURE 6C

FIGURE 7A

FIGURE 7B

FIGURE 7C

FIGURE 8

FIGURE 9

FIGURE 10

FIGURE   11

FIGURE 12

4 HOURS EXPOSURE TO 4°C

PERCENTAGE OOCYTES WITH NORMAL MEMBRANE POTENTIAL AFTER HYPOTHERMIC EXPOSURE

VALUES ARE MEAN + ONE STD DEVIATION

EACH GROUP IS 5 OOCYTES n is the number of groups.

| PBS | 0.1mg/ml AFGP1-8 | 1.0mg/ml AFGP1-8 | 40mg/ml AFGP1-8 | 40mg/ml AFGP1-5 | 40mg/ml AFGP7,8 |

n = 10, n = 6, n = 6, n = 14, n = 12, n = 10

EP 0 511 317 B1

FIGURE 13

24 HOURS EXPOSURE TO 4°C

PERCENTAGE OOCYTES WITH NORMAL MEMBRANE POTENTIAL AFTER HYPOTHERMIC EXPOSURE

| PBS | 0.1 mg/ml AFGP1-8 | 1.0 mg/ml AFGP1-8 | 40 mg/ml AFGP1-8 | 40 mg/ml AFGP1-5 | 40 mg/ml AFGP7,8 |

n = 4, 0%, P < 0.0005

n = 4, 0%, P < 0.0005

n = 4, P < 0.05

n = 4, P < 0.05

n = 4, 0%, P < 0.0005

n = 4, 0%, P < 0.0005

EP 0 511 317 B1